# EUROPÄISCHE PATENTSCHRIFT

(11) **EP 0 648 500 B1**
(45) Veröffentlichungstag und Bekanntmachung des Hinweises auf die Patenterteilung: **18.11.1998**
(21) Anmeldenummer: 93113005.8
(22) Anmeldetag: 13.08.1993
(51) Int. Cl.: A61K 38/41

(54) **Kardiotropes Mittel auf der Basis von Glykosid und einem beta-adrenergischen Reizmittel**
Cardiotrophic composition based on glycosides and beta-adrenostimulants
Composition cardiotropique basé sur des glycosides et des stimulants bêta-adrénorgiques

(43) Veröffentlichungstag der Anmeldung: 19.04.1995
(73) Patentinhaber: Karsanov, Nikolai Vasilievich, Tbilisi (GE)
(72) Erfinder: Karsanov, Nikolai Vasilievich, Tbilisi (GE); Kipshidze, Nodar Nikolaevich, Tbilisi (GE); Sukoyan, Galina Victorovna, Rustavi (GE); Khugashvili, Zinaida Grigorievna, Gldansky massiv (GE); Tatulashvili, Dali Robertovna, plato Nutsubidze (GE); Selikhova, Evgenia Vasilievna, Tbilisi (GE); Guchua, Eteri Ivlianovna, Digomsky massiv (GE)
(74) Vertreter: Patentanwälte Zellentin & Partner

(56) Entgegenhaltungen:
- FR-A- 2 009 148
- CHEMICAL ABSTRACTS, vol. 79, no. 15, 15. Oktober 1973, Columbus, Ohio, US; abstract no. 87428, GREEFF K. ET AL. 'Interaction between cardiac glycosides and beta-sympathomimetic drugs' Seite 20 ;Spalte 1 ; & ARZNEIM. -FORSCH., Bd.23, Nr.6, 1973 Seiten 759 - 764
- CHEMICAL ABSTRACTS, vol. 87, no. 24, 12. Dezember 1977, Columbus, Ohio, US; abstract no. 189353, BONELLI J. & HRUBY K. 'The bioavailability of beta-acetyldigoxin alone and combined with oxyfedrine' Seite 304 ;Spalte 1 ; & INT. J. CLIN. PHARMACOL. BIOPHARM., Bd.15, Nr.6, 1977 Seiten 288 - 293
- CHEMICAL ABSTRACTS, vol. 90, no. 25, 18. Juni 1979, Columbus, Ohio, US; abstract no. 197653, KIPSHIDZE N. ET AL. 'Effect of oxyfedrine and beta-acetyldigoxin on adenyl system nucleotide content and contractile properties of the bundles of glycerinated myocardial fibers in allergic and adrenaline myocarditis' Seite 45 ;Spalte 1 ; & DRUGS EXP. CLIN. RES., Bd.4, Nr.4, 1978, Seiten 127 - 136

## Beschreibung

Die vorliegende Erfindung betrifft die Kardiologie und bezieht sich insbesondere auf die Entwicklung von kardiotropen Arzneimitteln, welche effektive pharmakologische Wirkungen bei schweren und refraktären Formen der Herzinsuffizienz besitzen.

Als Prototyp des erfindungsgemässen Mittels, welches im weiteren auch als REFRAKTERIN bezeichnet wird, ist ein bekanntes kombiniertes pharmakologisches Mittel, nämlich Ildamen-novodigal (s. die PS Nr. 3332, Arzneim.-Forsch. (Drug Res.) 25 Nr. 3 (1975), F. Stroman und R. Hempel. Kombination von Oxyfedrin mit β-Acetyldigoxin) gewählt wirden.

Das erwähnte Ildamen-novodigal enthält 0,2 mg β-Acetyldigoxin und 18 mg Oxyfedrin, wird zur Behandlung von Herzinsuffizienzen, darunter auch einer sich durch Störungen des Blutkreislaufs entwickelnden Herzinsuffizienz angewendet. Je nach Erkrankungsschwere können die Tagesdosen auf 0,6 mg β-Acetyldigoxin und 36 mg Oxyfedrin gesteigert und die Behandlungsdauer auf einen Monat verlängert werden. Die Wirkung der Kombination von β-Acetyldigoxin mit Oxyfedrin beruht auf einem positiven inotropen Synergismus. Durch die Kombination von β-Acetyldigoxin mit Oxyfedrin werden die Toxizität von β-Acetyldigoxin wesentlich verringert, die Entwicklung der Bradykardie und Arrhythmie gehemmt und damit die Verträglichkeit des Präparats verbessert. Trotz einer Vergrösserung der Distanz zwischen therapeutischer und toxischer Dosis bei einer Kombination von β-Acetyldigoxin mit Oxyfedrin bleibt jedoch der Abstand zwischen diesen immer noch gering. Es kann auch bei der genannten Kombination zu einer Entwicklungsgefahr von Intoxikationserscheinungen, besonders bei einer erhöhten Empfindlichkeit des Herzens gegen Herzglykoside im Falle entzündlicher Schädigungen des Herzmuskels, besonders bei grippösen Schädigungen desselben sowie idiopathischen und alkoholischen Kardiomyopathien kommen.

Durch Anwendung der Kombination von β-Acetyldigoxin mit Oxyfedrin sogar in einer beträchtlich geringeren Dosis als bei Ildamen-novodigal ergibt sich bei einem Energiemangel-Zustand des Kardiomyozyts, z.B. einer infolge toxisch-allergischer Myokarditis aufgetretenen ausgeprägten Herzinsuffizienz eine übernormale Verstärkung der Kontraktionsaktivität des Systems kontraktiler Eiweisse des Myokards (vgl. Tabelle 2) welche zu einer Vergrösserung des Energiemangels im Kardiomyozyt (s. Tabelle 3) führt.

Bei getrennter Einwirkung dieser Verbindungen wird die Verstärkung der Kontraktionsaktivität des Systems kontraktiler Eiweisse auch von einer wesentlichen Erhöhung des Gehaltes an Adenosintriphosphorsäure (ATP) begleitet. Jedoch wird auch in diesem Fall der Gehalt an Creatinphosphat (CrP) immer noch vermindert (s. Tabelle 3).

Im Ergebnis einer Verschlechterung des metabolischen Zustandes geschieht eine Verstärkung dystrophischer Erscheinungen im Myokard, Kardiomyozyt (s. N.N. Kipshidze u.a., 1983, Materialy nauchnoi sessii NII klinicheskoi i eksperimentalnoi terapie Minzdrava Gruzii). Diese Erscheinung liegt, wie man anzunehmen hat, gerade dem refraktären Zustand des Herzens bei herkömmlichen Behandlungsverfahren zugrunde.

Aus der FR-A-2,009,149 sind herzwirksame Mittel mit β-Rezeptorwirkung zur Behandlung von Angina pectoris bekannt, welche zusätzliche Herzglykoside und Vasodilatatoren enthalten können, wodurch die Sauerstoffversorgung des Myokards verbessert und der negative isotrope Effekt der β-Blocker unterdrückt wird.

Aus CA 79(15) : 87 428 (Greeff, K. et al, Arzneim.-Forsch. 1973 23(6), 759-64) ist eine Interaktion zwischen Herzglykosiden und β-Sympathomimetika bekannt, durch die die Giftigkeit der Herzglykoside stark erhöht wird. Durch Vorbehandlung mit Reserpin kann dieser Effekt aufgehoben werden.

Aus CA 87(24) : 189 353 (Bonelli, J. et al, Int. J. Clin. Pharmacol. Biopharm. 1977, 15(6), 288-293) ist bekannt, daß die Bioverfügbarkeit von Novodigal und Ildamen-Novodigal-Kombination praktisch gleich ist.

CA 90(25): 197,653c (Kipshidze, N. et al, Drugs Exp. Clin. Res. 1978, 4(4), 127-136) beschreibt die Wirkung von Oxyfedrin und β-Acetyl-digoxin und ihrer Kombination bei Myokarditis. Untersucht wurde die Energieversorgung (ATP) und Kontraktibilität von glycerinierten myocardialen Fasern bei Kaninchen.

Die Erfindung bezweckt die Entwicklung eines kardiotropen Mittels, dessen pharmakologische Wirkung bei geringer Toxizität die Möglichkeit bietet, die Kontraktilität wiederherzustellen und die Kraftreserven im Myokard bei einer ausgeprägten und schweren Herzinsuffizienz, darunter bei einer refraktären Insuffienzform gegenüber den herkömmlichen Behandlungsverfahren wie Ildamen-Novodigal zu erhöhen sowie die Zeiten für das Erreichen einer positiven pharmakologischen Wirkung zu verkürzen.

Dies wird dadurch erreicht, daß das enfindungsgemäß entwickelte Mittel, im weiteren als REFRAKTERIN bezeichnet, zum Unterschied von Ildamen-Novodigal zusätzlich Nikotinamid-Adenin-Dinukleotid (NAD), Zytochrom C und Inosin (Riboxin) umfasst sowie Oxyfedrin und β-Acetyldigoxin in wesentlich kleineren Dosen gegenüber Ildamen-novodigal enthält, wobei die Komponenten in einem folgenden Massenverhältnis zueinander (in Gew.-Teilen)

| | |
|---|---|
| Herzglykosid | 0,05 bis 0,2 |
| β-Sympathomimetikum (β-adrenergisches Reizmittel) | 0,3 bis 3,5 |
| Nikotinamid-adenin-dinukleotid | 0,5 bis 5 |
| Zytochrom C | 5 bis 15 |
| Inosin (Riboxin) | 20 bis 250 |

genommen werden.

Aus unseren fundamentalen Forschungen folgt, dass der schweren Hierzinsuffizienz - in einer weit gegangenen Phase ihrer Entwicklung bei verschiedenen Herzerkrankungen sowie bei deren refraktären Formen, die sich bei entzündlichen Schädigungen des Myokards, alkoholischer Kardiomyopathie (und offensichtlich bei anderen Erkrankungen) entwickeln- eine gleichzeitige Schädigung von drei Subzellensystemen hauptsächlich zugrunde liegt, die für die Aktion Kontraktion-Erschlaffung des Kardiomyozyts verantwortlich sind und zu denen ein System kontraktiler Eiweisse, ein System zum Transport von Ca durch Membranen (welches die Reizung mit der Kontraktion koppelt und die Kraft der Kontraktionsantwort regelt sowie die Erschlaffung des Myokards bewirkt) und ein System zur Energieversorgung (das alle Systeme der Zelle mit einer Quelle verwendbaren, leicht zugänglichen Energie versorgt) gehören ( vgl. Tabelle 1).

Ausgehend davon muss zur Überwindung des refraktären Zustandes und zur erfolgreichen Wiederherstellung der Kontraktionstätigkeit des Herzens die normale Funktion aller drei genannten Systeme der Zelle harmonisch wiederhergestellt werden, weil -wie schon erwähnt - die Stimulierung eines (oder zweier Systeme, wie es bei Ildamen-Novodigal der Fall ist) von diesen drei Systemen zu keiner Normalisierung derKontraktionstätigkeit der Zelle - des Herzens - führt, sondern sich die Erscheinung einer Unempfindlichkeit gegen das anzuwendenden Mittel abzeichnet. Vielmehr kann eine Verschlechterung des strukturellfunktionellen Zustandes des Kardiomyozyts wegen eines grossen (unauffüllbaren) Verbrauch an ATP, d.h. einer Vergrösserung des Energiemangels eintreten.

Das Problem zur Überwindung des refraktären Zustandes und zur schnellen erfolgreichen Wiederherstellung der Funktionsaktivität aller drei Systeme des Kardiomyozyts läuft also auf die harmonische Wiederherstellung der funktionellen Arbeitsfähigkeit aller drei Systeme der Myokardialzelle hinaus,die für die Aktion Kontraktion-Erschlaffung verantwortlich sind. Dabei ist die adäquate Wiederherstellung (in bezug auf die Leistung) der Aktivität der Energieversorgungssysteme von vorherrschender Bedeutung, weil sowohl die Erhöhung der Funktionsaktivität des Systems kontraktiler Eiweisse als auch die Aktivierung der Funktion der Ionenpumpen eine wesentliche Vergrösserung der Menge der von der Zelle aufgenommenen Energie nach sich zieht. Gerade der Mangel an Energie liegt voraussichtlicht dem refraktären Zustand schwerer Formen der Herzinsuffizienz in Anwendung auf die herkömmlichen Behandlungsverfahren zugrunde, bei denen in der Regel eine einseitige Stimulation des Systems kontraktiler Eiweisse ohne entsprechende Verstärkung der Energieversorgung der Zelle geschieht.

Der Energiemangel entwickelt sich bei sämtlichen Herzerkrankungen, die von der Entwicklung eines hypoxischen Zustandes des Myokards (Infarkt des Herzmuskels, entzündliche und alkoholische Schädigungen des Herzens, Herzinsuffizienz in der terminalen Phase u.a.) begleitet werden, nicht nur durch die Verminderung der Beförderung des Sauerstoffes in die Zelle sondern auch im Zusammenhang damit, dass die Zelle einen beträchtlichen Anteil an Zytochrom C, NAD und Adenylnukleotiden verliert.

Mit dem erfindungsgemäss entwickelten, kombinierten Präparat, das NAD, Zytochrom C, Inosin, Herzglykosid (β-Acetyldigoxin oder β-Methyldigoxin oder Strophantin K, ein Reizmittel für das System kontraktiler Eiweisse) und ein β-adrenergisches Reizmittel (Oxyfedrin oder Nonachlasin, β-Sympathomimetika kardiotroper Wirkung) enthält, gelang es, das Problem zur Überwindung des refraktären Zustandes der Herzinsuffizienz und zur schnellen Wiederherstellung des strukturell-funktionellen Zustandes des Myokardes zu lösen.

Die Anwendung dieses Präparats bei einer toxischallergischen Myokarditis, die durch die Entwicklung einer ausgeprägten Insuffizienz der Kontraktionstätigkeit des Herzens gekennzeichnet wird, gewährleistet eine vollständige Wiederherstellung (vgl. Tabelle 4) der intrakardialen und peripherischen Hämodynamik sogar bei den Bedingungen einer durch den Druck (s. Tabelle 5) hervorgerufenen Überlastung des Herzens.

Ein solcher Effekt wird durch harmonische Wiederherstellung der funktionellen Arbeitsfähigkeit aller drei Systeme des Kardiomyozyts erzielt, die für die Aktion Kontraktion-Erschlaffung verantwortlich sind.

Dabei entfaltet sich ein folgendes Bild:

Das exogene NAD deckt das Defizit eines Zytosol- und eines Mitochondrialpools von NAD ab. Dies stellt die Aktivität der glykolytischen NAD-anhängigen Dehydrogenasen, die glykolytische Oxydoreduktion und dadurch die Synthese von ATP auf glykolytischem Wege wiederher sowie stellt die Intensität des Transportes des Zytosol-Protons in der Mitochondrie (über das Malat-Asparat-Shunt) zu dessen weiterer Verwendung im Elektronenübertragungskreis wiederher.

Durch das Auffüllen des Defizits des Mitochondrialpolls von NAD wird die Aktivität der NAD-abhängigen Dehydrogenasen des Krebszyklus stimuliert und der Strom von Protonen aus dem Zyklus zum Elektronenübertragungskreis hin wiederhergestellt.

Seinerseits wird des exogene Zytochrom C in den Mitochondrien (in seine Stelle) im Elektronenübertragungskreis eingebaut und damit (dem Volumen nach) der Transport von Elektronen zum Sauerstoff hin, d.h. die normale ATP-synthesierende Fähigkeit der Mitochondrien in der Reaktion der oxydativen Phosphorilierung wiederhergestellt, die mit dem Durchlauf von Elektronen über deren Übertragungskreis verbunden ist.

Das geschilderte Bild kann sich in vollem Masse nur bei gleichzeitigem Auffüllen der Defizite an NAD und Zytochrom C abwickeln.

Durch das Auffüllen des Defizits an NAD allein ergibt sich keine (der Leistung nach) vollständige Wiederherstellung der Funktion des Energieversorgungssystems, weil der Durchsatz des Elektronenübertragungskreises niedrig bleibt und dieser Übertragungskreis mit den Strömen von Protonen aus dem Krebszyklus und dem Zytosol nicht zurechtkommt. Im Zusammenhang damit nimmt unter Einwirkung von exogenen NAD der Gehalt an Creatinphosphat auf die Norm (Tabelle 6) nicht zu, wobei der Gehalt an ADP eine Tendenz zur Erhöhung und das ATP/ADP-Verhältnis eine Tendenz zur Senkung (s. Tabelle 6 und 7) erwirbt.

Das Auffüllen des Defizits an Zytochrom C (das z.Z. weit verwendet wird) ergibt ebenfalls keine vollständige Wiederherstellung der ATP-synthesierenden Kapazität des Energieversorgungssystems (Tabelle 6), da das Auffüllen des Defizits an Zytochrom C zu keiner Wiederherstellung des normalen Verhältnisses von NAD zu NAD.H (Tabelle 8) führt, das die Aktivität der Dehydrogenasen reguliert. Der Gehalt an ADP fällt hierbei ab, während das [ATP]/[ADP] - Verhältnis die Norm übersteigt.

Weder das Auffüllen des Defizits an NAD (Tabelle 8) noch das Auffüllen des Defizits an Zytochrom C führt zur Normalisierung des gesamten Gehaltes an Adenylnukleotiden (Tabellen 6, 7). Er bleibt gering.

Die Wiederherstellung des gesamten Gehaltes an Adenylnukleotiden wird durch Einführung von Inosin in die Kombination erreicht, das die Synthese von Adenylnukleotiden de novo (Tabelle 9) anregt.

Inosin trägt ausserdem zu einer wesentlichen Verstärkung des Koronarkreislaufs (zu einer wesentlich stärkeren Beförderung des Sauerstoffes in den Myokard) bei und bewirkt, dass in der Peripherie Oxyhämoglobin leichter den Sauerstoff abgibt. Dies leistet bedinungslos einen wesentlichen Beitrag Zur Wiederherstellung der ATP-synthesierenden Fähigkeit (Kapazität) des Kardiomyozyts, was bei steigenden Belsatungen des Herzens wichtig ist.

Somit wird durch Einführung von exogenen NAD, Zytochrom C und Inosin in die Kombination, welche das Defizit an endogenen NAD und Zytochrom C und offensichtlich Inosin auffüllen, die vollständige Wiederherstellung der Homeostasis des Energieversorgungssystems (Gehalt an ATP, ADP, AMP, CrP, NAD und NAD.H) und dessen funktionelle Fähigkeit gewährleistet. Da für die Regulation des Energieversorgungssystems nicht nur der absolute Gehalt an ATP, ADP, AMP, CrP, NAD und NAD.H sondern auch deren Verhältnisse, namlich [ATP]/[ADP], [ADP]·[Cr]·[P_{anorgan.}]/[ATP]·[CrP] , von Bedeutung ist, handlet es sich erfindungsgemäss nicht um eine blosse Summation von Effekten (jedes der zu verwendenden Mittel aktiviert in bestimmten Masse die Synthese von ATP), sondern um die Wiederherstellung der feinen Regulation des Systems. Hierbei ist zu beachten, dass die erreichbare Normalisierung des Gehaltes an ATP und ADP für den strukturellen Zustand der kontraktilen Eiweisse ebenfalls von grosser Bedeutung ist.

Als nächste Aufgabe, die nach der Wiederherstellung der Funktion des Energieversorgungssystems folgt, ist bei der Behandlung der Herzinsuffizienz die Wiederherstellung der verlorenen Fähigkeit des Systems kontraktiler Eiweisse, die Kraft zu generieren (Tabelle 10), zu bezeichnen. Diese Aufgabe ist durch Einführung eines Herzglykosids (Tabelle 10) in die Kombination, welches einen unmittelbaren Einfluss auf das System kontraktiler Eiweisse ausübt, gelöst. Im kombinierten Präparat REFRAKTERIN als Basismittel ist β-Acetyldigoxin benutzt.

Jedoch ist es bei einer erhöhten Empfindlichkeit des Herzens gegen Herzglykoside (z.B. bei enzündlichen Schädigungen des Herzens, bei idiopatischer Kardiomyopatie) zweckmässig, β-Acetyldigoxin durch β-Methyldigoxin (Refrakterin BM) zu ersetzen, weil die spezifische Aktivität von β-Methyldigoxin die von β-Acetyldigoxin um zwei Grössenordungen übersteigt (die optimalen Konzentrationen betragen 10⁻⁸bzw. 10⁻⁶ M (Tabelle 11)).

Bei einer akuten Herzinsuffizienz, die sich auf dem Hintergund des Energiemangels entwickelt, wird zweckmässigerweise an Stelle von β-Acetyldigoxin Strophantin - K (Refrakterin CK) verwendet, das, genommen in einer optimalen Dosis, auf die Energieumwandlung nicht nur eine quantitative Einwirkung (wie β-Acetyldigoxin), sondern auch eine qualitative Einwirkung (Tabelle 11, 14) ausübt, d.h. die Wirtschaftlichkeit des Kontraktionsvorganges erhöht. Hinzu kommt, dass der Bereich der aktiven Konzentrationen von Strophantin K den gesamten Bereich von β-Acetyl- und β-Methyldigoxin überstreicht. Dies macht es möglich, kleinere Konzentrationen von Glykosid als in REFRAKTERIN effektiv zu benutzen.

Da β-Methyldigoxin in einer optimalen Konzentration wie auch Strophantin K auf die durch das System kontraktiler Eiweisse vorgenommene Energieumwandlung nicht nur eine quantitative sondern auch eine qualitative Einwirkung ausübt, ist Refrakterin BM bei einer akuten und chronischen Herzinsuffizienz angezeigt, die auf dem Hintergrund einer Hypoxie, eines Energiemangels verläuft.

Schliesslich ist als letzte Aufgabe der Erfindung die Notwendigkeit der Wiederherstellung der normalen Funktion des Systems zum Transport von Ca (Tabelle 13) zu bezeichnen. Da die Herzglykoside der Fingerhut-Gruppe (zum Unterschied von Strophantin G) auf das System kontraktiler Eiweisse keinen unmittelbaren Einfluss ausüben (Strophantin K wirkt auf beide Systeme ein), ist zur Wiederherstellung des funktionellen Zustandes des Systems zum Transport von Ca in das kombinierte Basispräparat (REFRAKTERIN) ein β-adrenergisches Reizmittel kardiotroper Wirkung-Oxyfedrin-eingeführt. Mit Oxyfedrin wird die Aufnahme und der Auswurf von Calzium aus dem sarkoplasmatischen Retikulum stimuliert und die Intensität dieser Vorgänge auf die normalen Werte (Tabelle 14) gebracht. Darüber hinaus wird durch Oxyfedrin auf das System kontraktiler Eiweisse eingewirkt und die Intensität der Glykolyse durch Überführung der Phosphorylase in die aktive Form aktiviert.

All diese Wirkungen übt Oxyfedrin durch die Aktivierung von Adenylatzyklase (Tabelle 14a) und durch Bedrücken der der Aktivität der Phosphodiesterase (Tabelle 14b) sowie dank einer Vergrösserung von AMP im Myokard aus.

Oxyfedrin übt auch auf das Energiebildungssystem einen Einfluss aus, d.h. erhöht den Gehalt an ATP, wobei der Gehalt an CrP aber wesentlich vermindert wird (Tabelle 3).

Oxyfedrin kann durch ein analoges Mittel - Nonachlasin (REFRAKTERIN H) - ersetzt werden, wobei jedoch die Dosis von Nonachlasin um mindestens das 5-fache (Tabelle 14c) gesteigert werden muss.

Jeder der Bestandteile des erfindungsgemässen REFRAKTERIN erhöht in dem einen oder anderen Mass somit den Gehalt an ATP, vergrössert die Kontraktilität des Systems kontraktiler Eiweisse, verstärkt den Transport von Ca über die Membranen des Kardiomyozyts. Das Endeffekt des erfindungsgemässen REFRAKTERIN stellt jedoch keine Summe von einzelnen Effekten dar, sondern ist ein Resultat eines komplizierten Regelung der Funktionsaktivität aller drei Systeme des Kardiomyozyts, welche für die Aktion Kontraktion - Erschlaffung verantwortlich sind, durch die die Kontraktionstätigkeit des Kardiomyozyts (sogar bei erhöhten Belastungen) vollständig normalisiert wird.

Man stellte das erfindungsgemässe Mittel wie folgt her. 0,5 mg NAD, 10 mg Zytochrom C, 80 mg Inosin, 0,3 mg Oxyfedrin und 0,075 mg β-Acetyldigoxin (berechnet auf kg Tiergewicht) vermischte man und löste in 6 ml einer auf 38 bis 40°C erwärmten physiologischen Lösung unter Schütteln unmittelbar vor dem Gebrauch. Das Präparat wurde von dem Auflösen bei 4°C gelagert.

### Beispiel 1. Pharmakologische Wirkung von Refrakterin bei toxisch-allergischer Myokarditis (TAM) von 3 tägiger Dauer

Man reproduzierte TAM bei Kaninchen nach der Methode von S.V Andreev und V.M. Sokolov (1968, "Sanogenez", "Meditsina"). Dazu wurden intravenös in einem Abstand von 4 Tagen zweimal je 2 ml Pferdeblutserum und 7 Tage nach der letzten Injektion 0,5 ml Staphylokokkentoxin injiziert.

Die pharmakologische Wirkung des erfindungsgemässen REFRAKTERIN registrierte man nach 5 tägiger Kur seiner Verabreichung in einer täglichen Dosis von 90,875 mg/kg, die ab dem zweiten Tag der Erkrankungsentwicklung begann.

Die zweitägige Verabreichung von REFRAKTERIN normalisierte bei TAM von 3 tägiger Dauer den Zustand der peripherischen und intrakardialen Hämodynamik, d.h. es wurden die systolische und diastolische Funktion des Herzenz (Tabelle 4) und die Koordination dieser Funktionen wiederhergestellt; es wurde die Kongestion in den Lungen und der Leber beseitigt. Dabei wurde der Funktionszustand des Kardiomyozyts so zuverlässig wiederhergestellt, dass das Herz sogar erhöhte Belastungen (Tabelle 5) überlebte.

### Beispiel 2. Pharmakologische Wirkung von REFRAKTERIN bei TAM von 10 tägiger Dauer

Man reproduzierte TAM wie auch bei TAM 3 tägiger Dauer.

Die pharmakologische Wirkung von REFRAKTERIN registrierte man nach 5 tägiger Verabreichung des erfindungsgemässen Präparats in einer täglichen Dosis von 90,875 mg/kg. In diesen Versuchen wurden zusätzlich zur Beeinflussung des Entzündungsprozesses den Tieren ab dem fünften Tag der Erkrankungsentwicklung gleichzeitig mit der parenteralen Verabreichung von REFRAKTERIN Aspirin® in einer täglichen Dosis von 0,25 mg/kg Tiergewicht peroral verabreicht. In speziellen Versuchen wurde dabei festgestellt, dass Aspirin® in dieser Dosis auf den Funktionszustand keines der drei Systeme des Kardiomyozyts, die für die Aktion Kontraktion-Erschlaffung verantwortlich sind, einen beachtlichen Einfluss ausübte.

Die tägliche Verabreichung von REFRAKTERIN während 5 Tage führte zur Beseitigung der Kongestion in der Peripherie (dabei fiel der Gehalt der Flüssigkeit in der Leber, den Lungen und dem Herzen selbst auf die Norm ab), zur Wiederherstellung der systolischen und diastolischen Funktion des Herzens, zu deren Koordination, wobei dies nicht nur im Ruhezustand (Tabelle 4) sondern auch unter den Bedingungen einer erhöhten Belastung des Herzens (Tabelle 5), was besonders wichtig ist, erreicht wurde.

Bei TAM von 10 tägiger Dauer fand unter Einfluss von REFRAKTERIN eine oordinierte Wiederherstellung der Funktionsaktivität des Systems kontraktiler Eiweisse (Tabelle 10), des Systems zum Transport von Ca über die Membranen des Kardiomyozyts (Tabelle 13) statt; bei adäquaten Stimulierung der Bildung einer zur Verwertung leicht zugänglichen Energie von ATP und einer Energie-reserve in Form von CrP (Tabelle 15) wird die Ultrastruktur des Kardiomyozyts erheblich verbessert. Dabei ist es nennenswert, dass die Wiederherstellung der Intensität des Kontraktionsvorganges und des Transportes von Ca bei der Bedingungen der Versorgung der Zelle mit Energie auch bei einer erhöhten Belastung des Herzens geschieht. Somit findet unter Einfluss von REFRAKTERIN eine stabile ausgegliche, harmonische Wiederherstellung der Funktion aller drei Systeme des Kardiomyozyts, die für die Aktion Kontraktion-Erschlaffung verantwortlich sind, statt.

### Vergleich der Wirkung von REFRAKTERIN mit der Wirkung einer Kombination von β-Acetyldigoxin mit Oxyfedrin

Es wird durch Verabreichung der Kombination von β-Acetyldigoxin mit Oxyfedrin in einer bedeutend geringeren Dosis als im Ildamen-novodigal - 0,15 mg/kg β-Acetyldigoxin und 0,6 mg/kg Oxyfedrin - während sowohl 5 Tage bei TAM 3 tägiger Dauer als auch während 5 Tage bei TAM 10 tägiger Dauer eine sprunghafte Erhöhung der durch das System kontraktiler Eiweisse des Myokards generierten Spannung (Tabelle 16) hervorgerufen, während der Gehalt an Adenylnukleotiden auf dem Niveau bleibt, das in der Kontrolle zu verzeichnen ist. Aber der Gehalt an CrP bleibt sowohl bei TAM 3 tägiger Dauer als auch bei TAM 10 tägiger Dauer auf dem Niveau des Kontrollwertes erhalten. Bei allergischer Myokarditis und einer Adrenalin-Koffein-Schädigung des Herzens und bei Anwendung dieser Kombination in einer noch geringeren Dosis - 0,05 mg/kg β-Acetyldigoxin und 0,2 mg/kg Oxyfedrin - führt die Zunahme der durch das System kontraktiler Eiweisse generierten Spannung zur Senkung des Gehaltes nicht nur an CrP sondern auch an ATP.

Bei Anwendung von REFRAKTERIN wird die Vergrösserung des Wertes der durch das System kontraktiler Eiweisse generierten Spannung von einer angemessenen Zunahme der Intensität des Transportes von Ca über die Membranen des Kardiomyozyts und der Produktion einer bei der Kontraktion leicht verwertbaren Energie begleitet.

Somit wird durch die Wirkung von Ildamen-Novodigal bei Myokarditiden, sogar bei kurzzeitiger Einnahme der Energiemangel-Zustand des Kardiomyozyts vertieft und letzten Endes eine Verschlechterung des strukturellen Zustandes des Myokards, d.h. eine Verstärkung von Erscheinungen der parenchymatösen Dystrophie hervorgerufen.

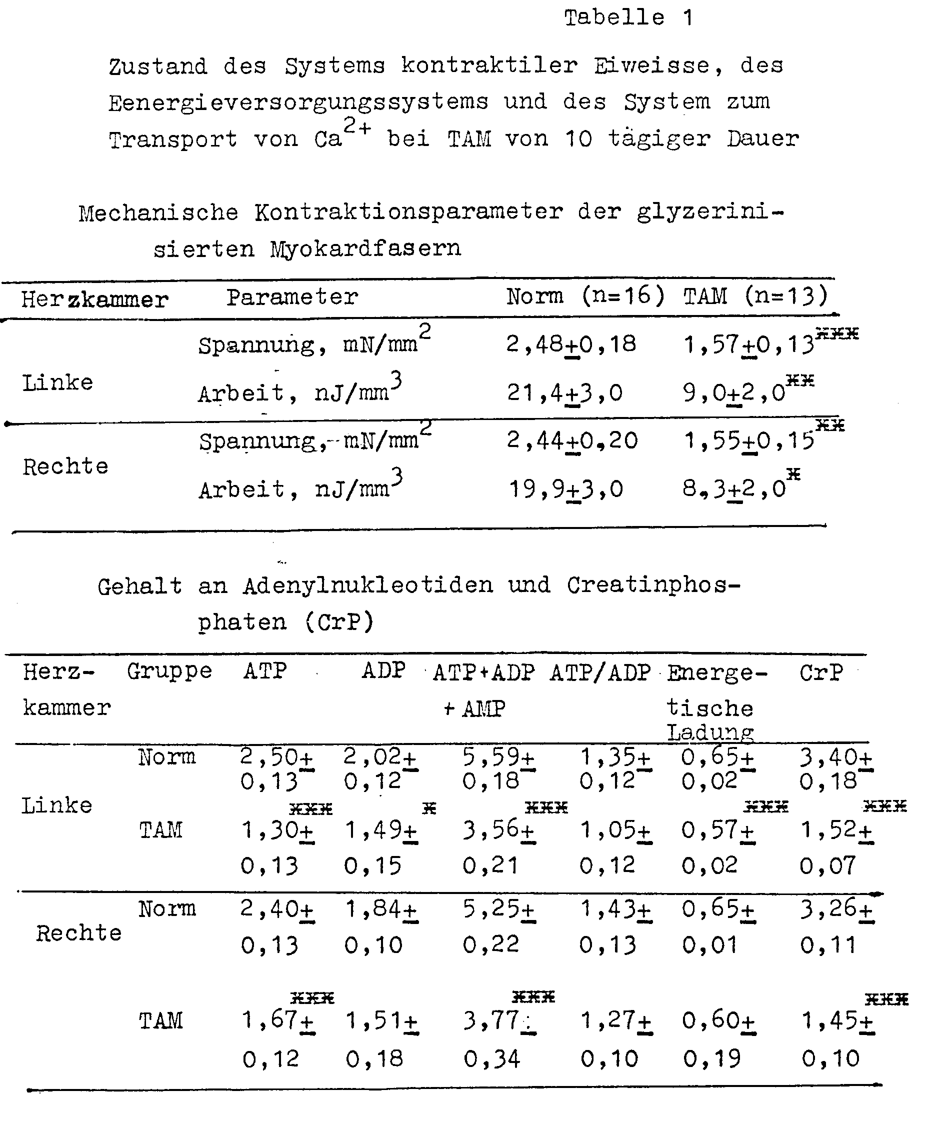

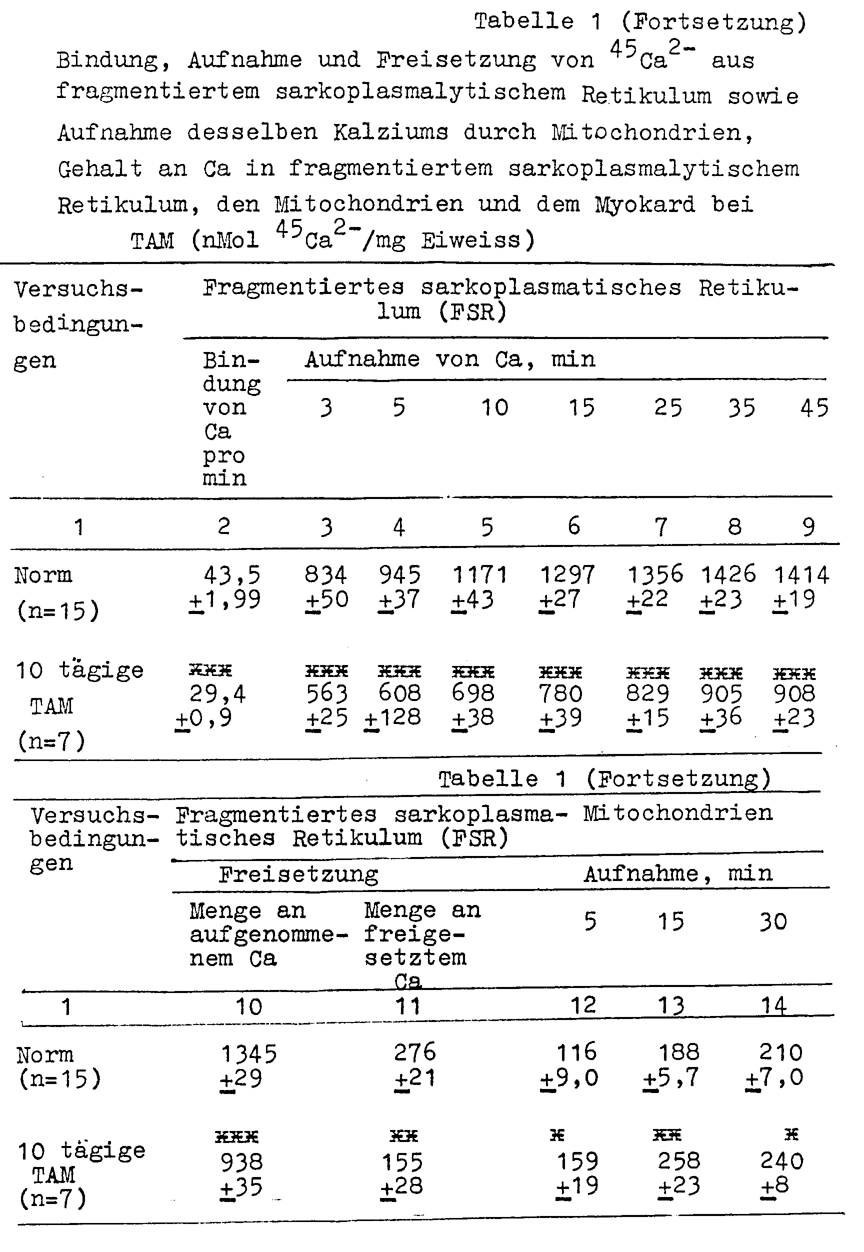

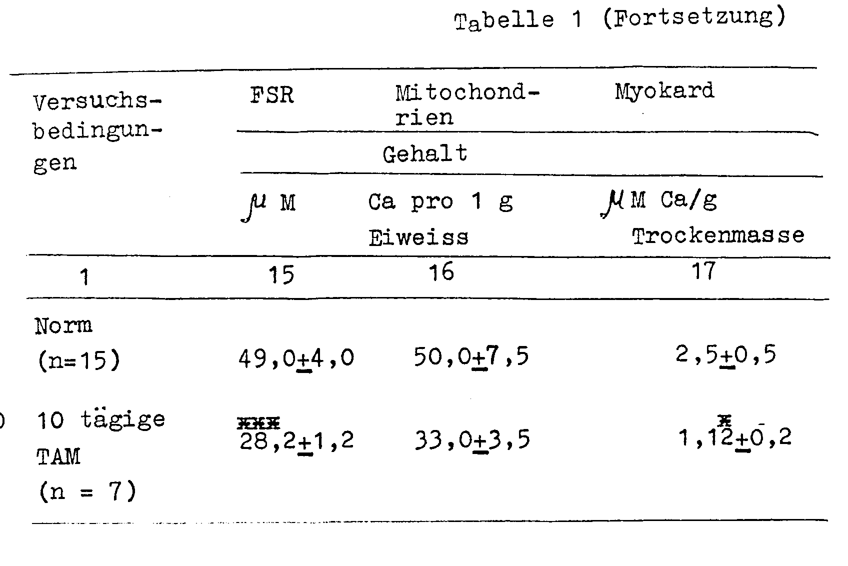

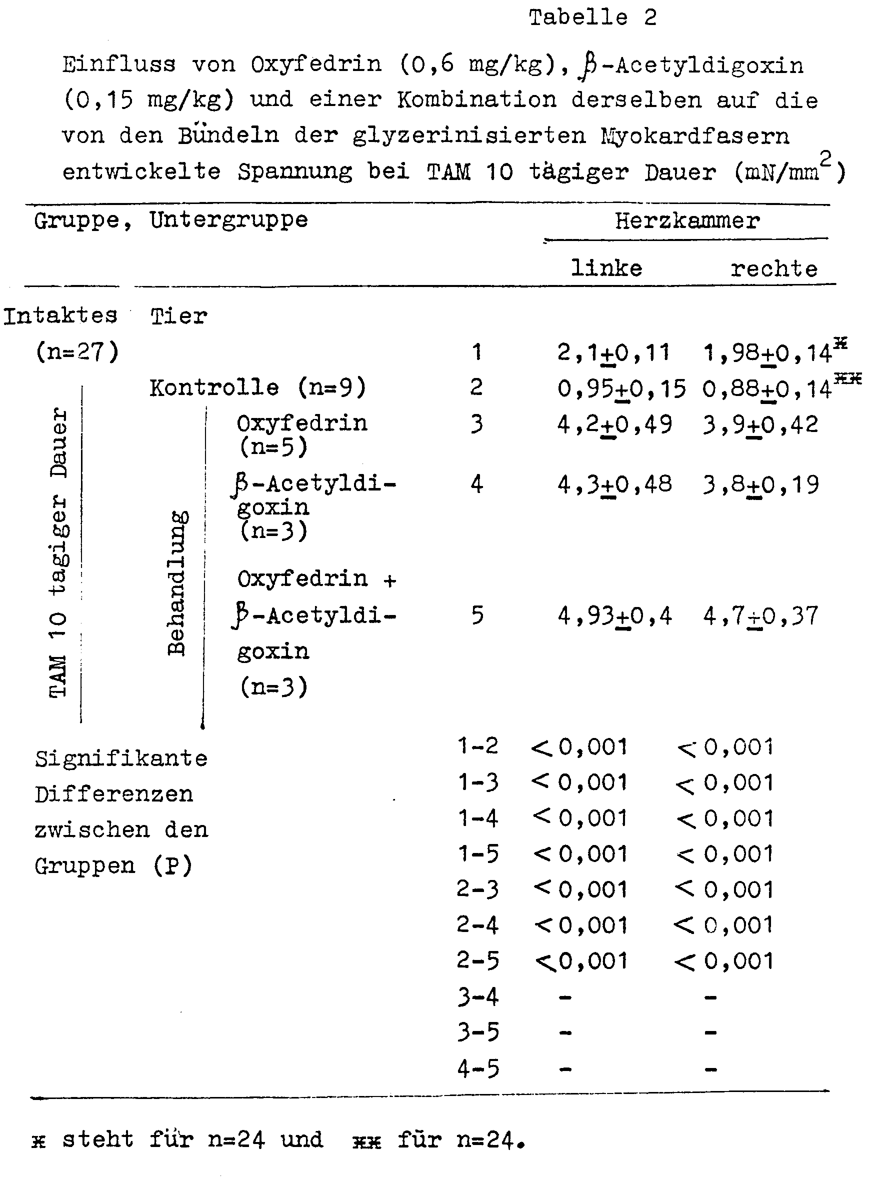

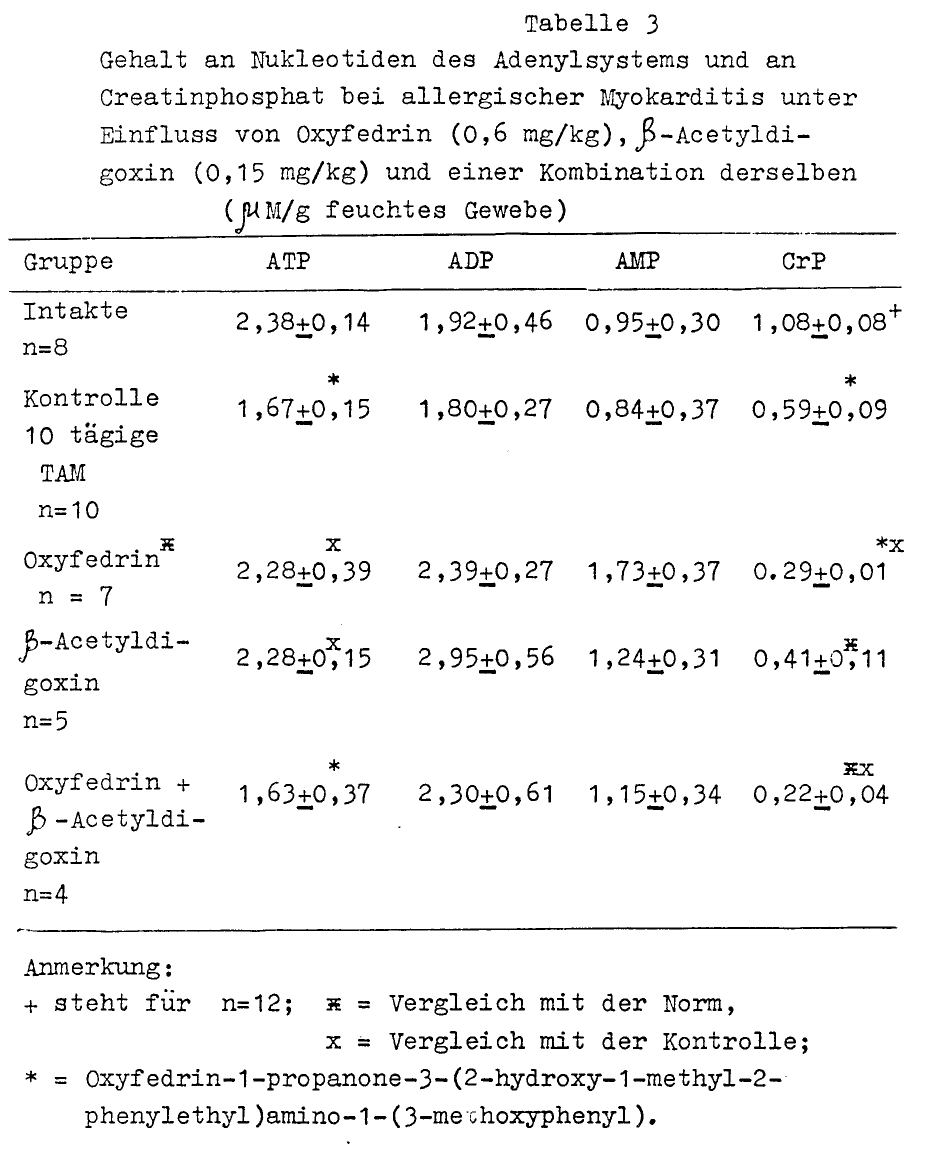

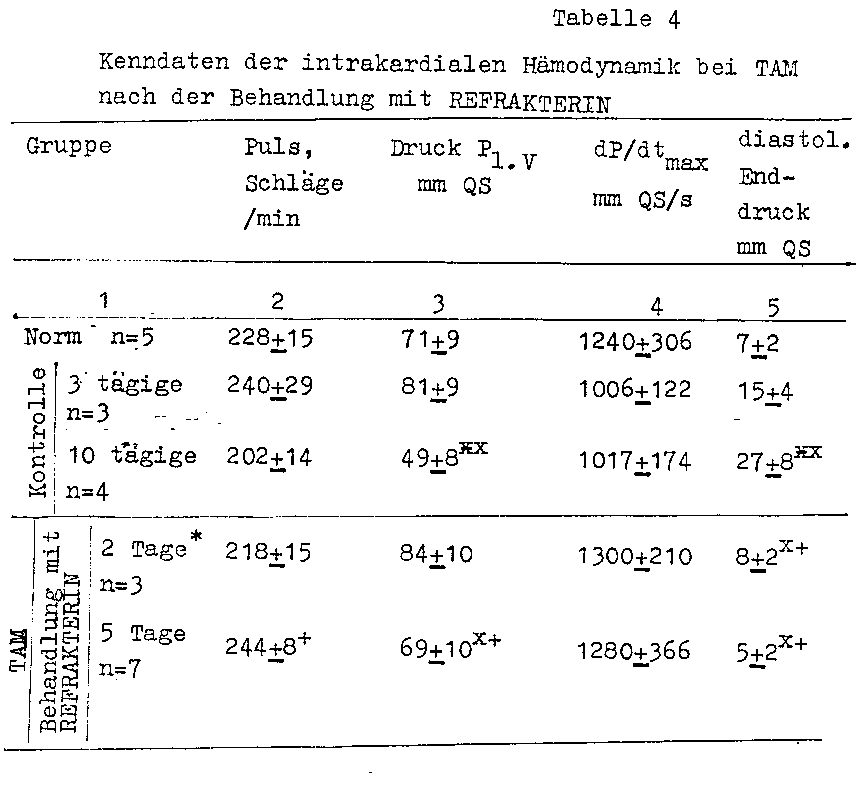

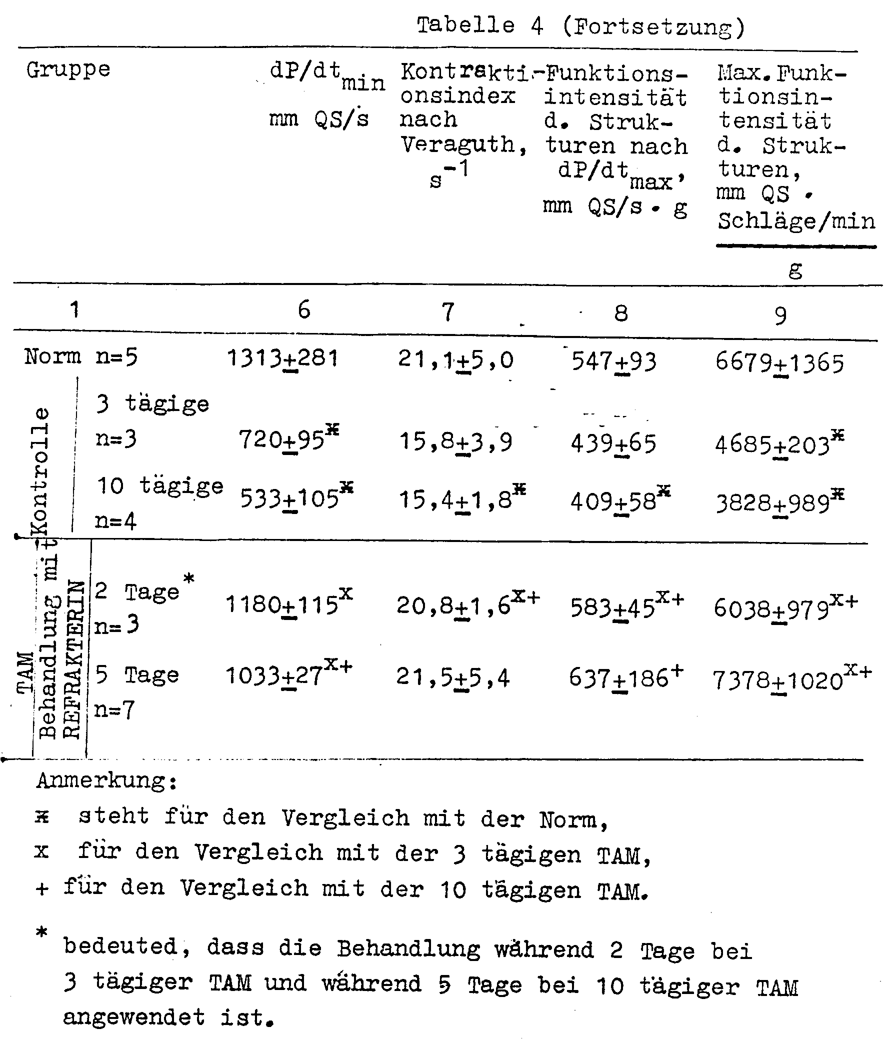

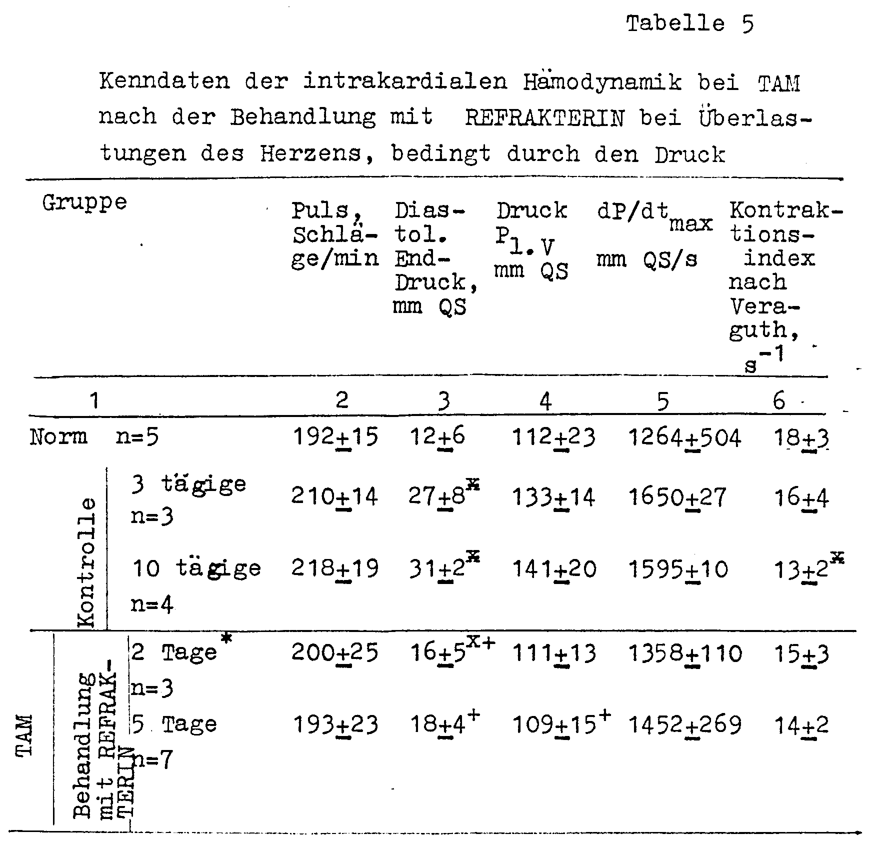

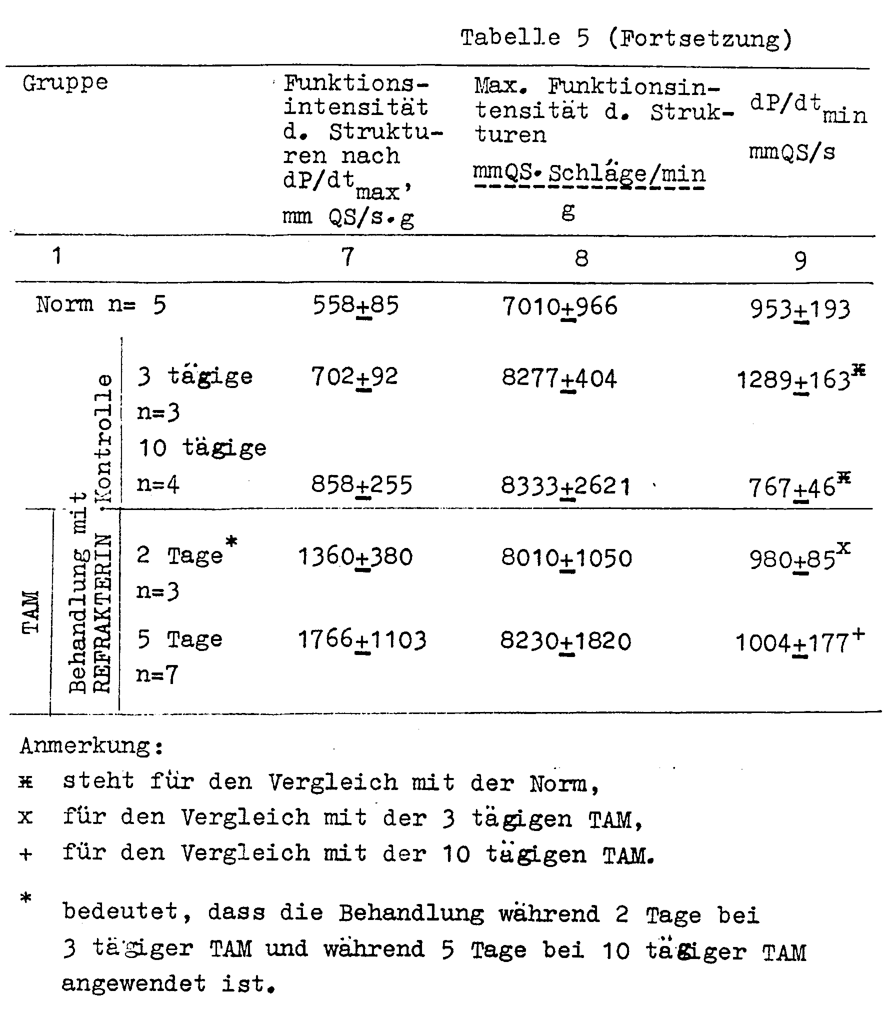

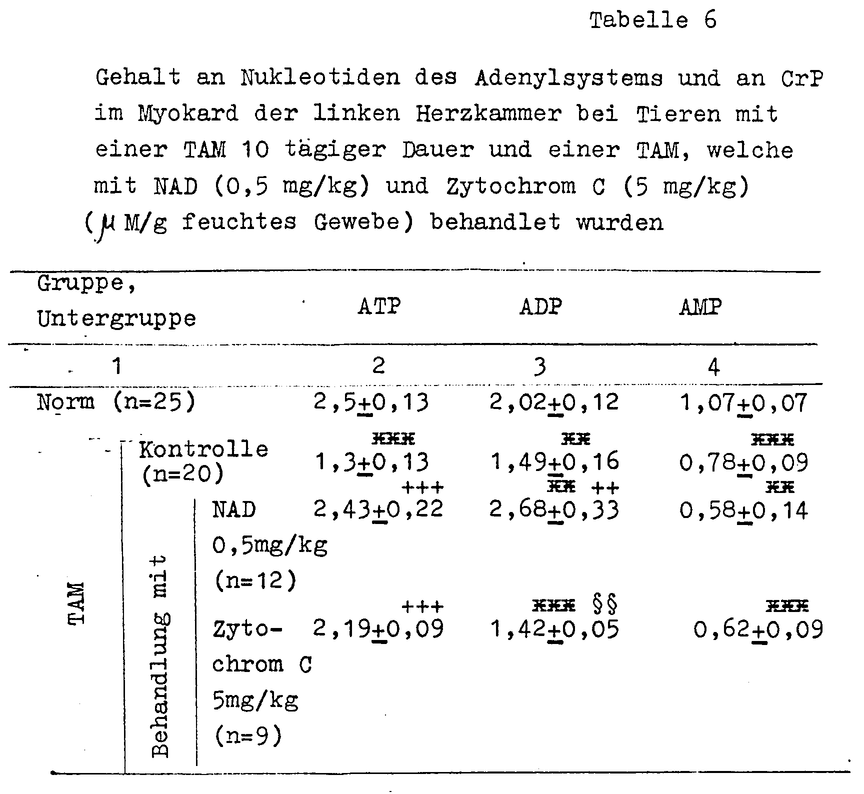

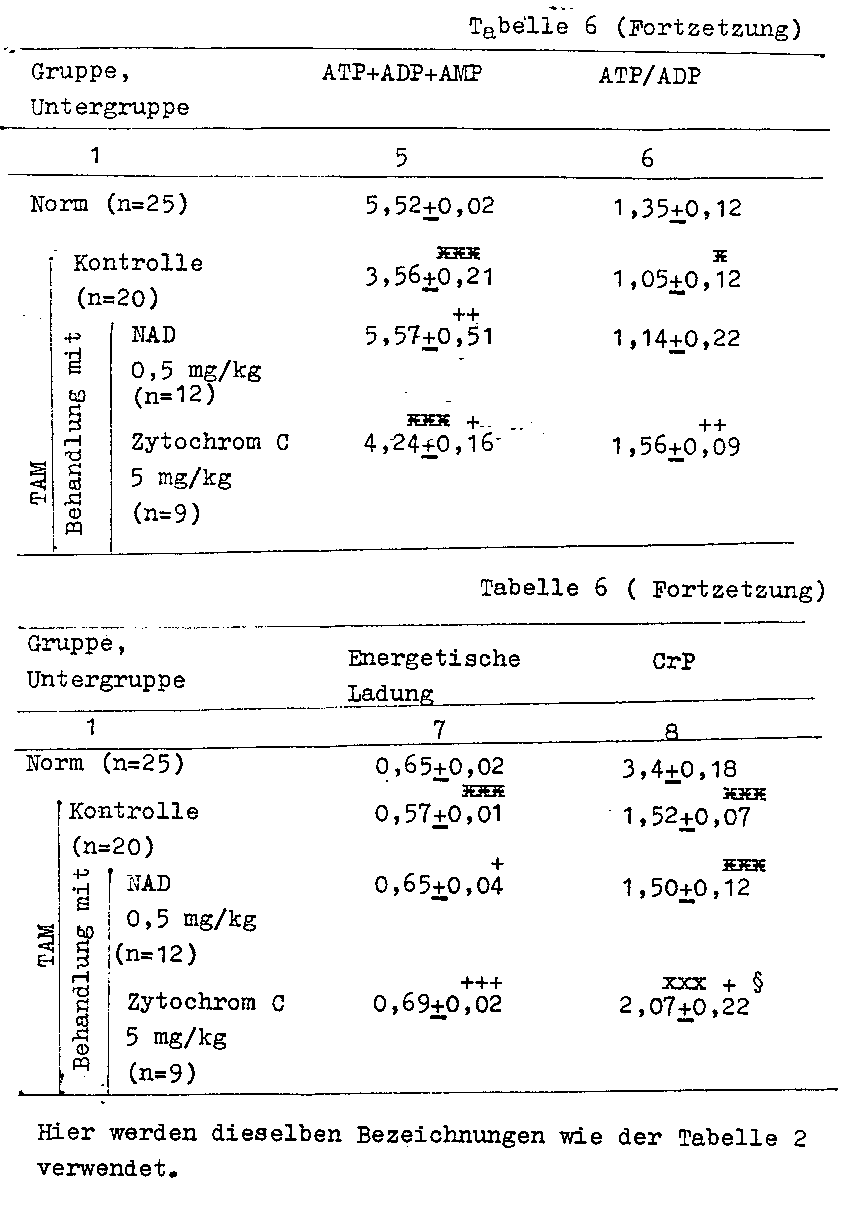

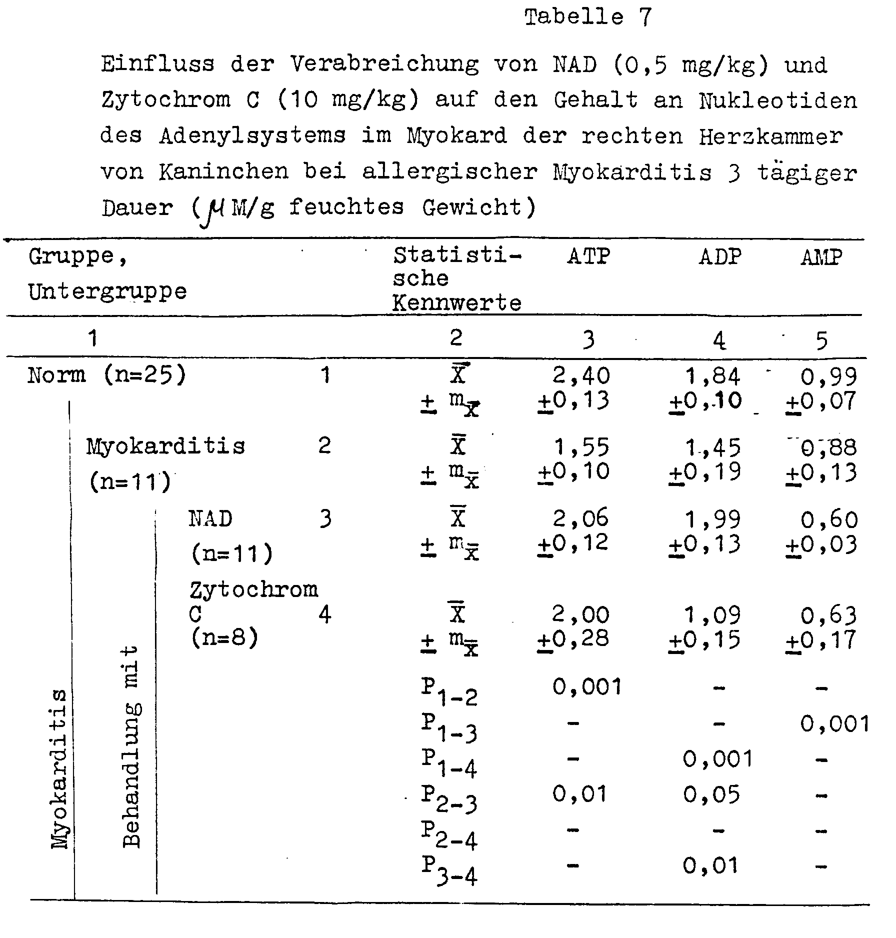

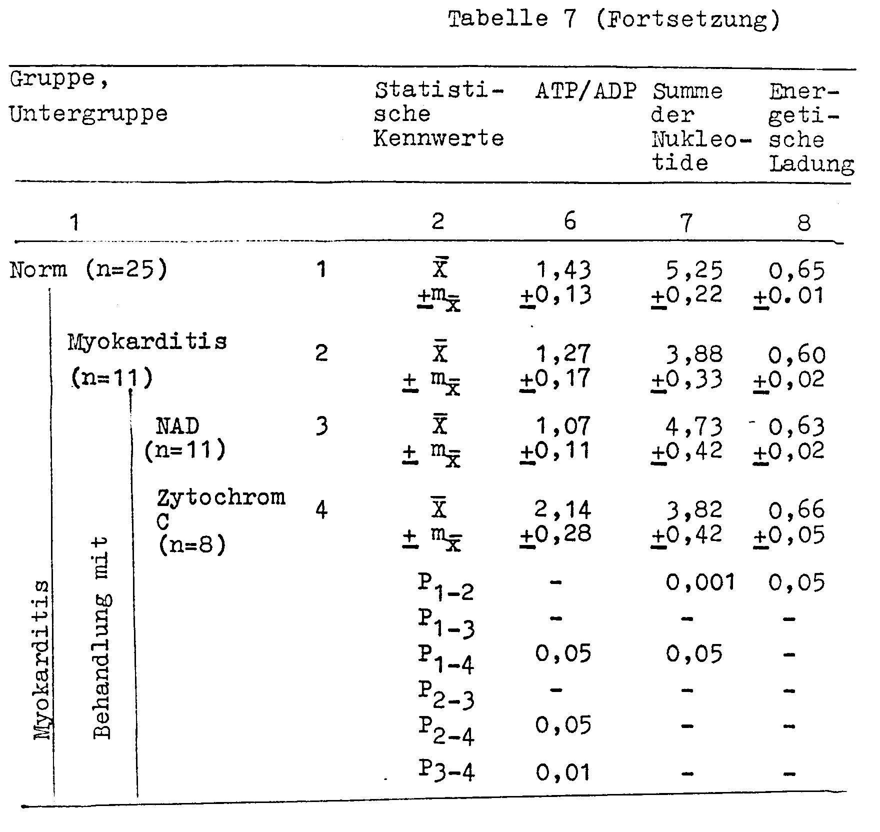

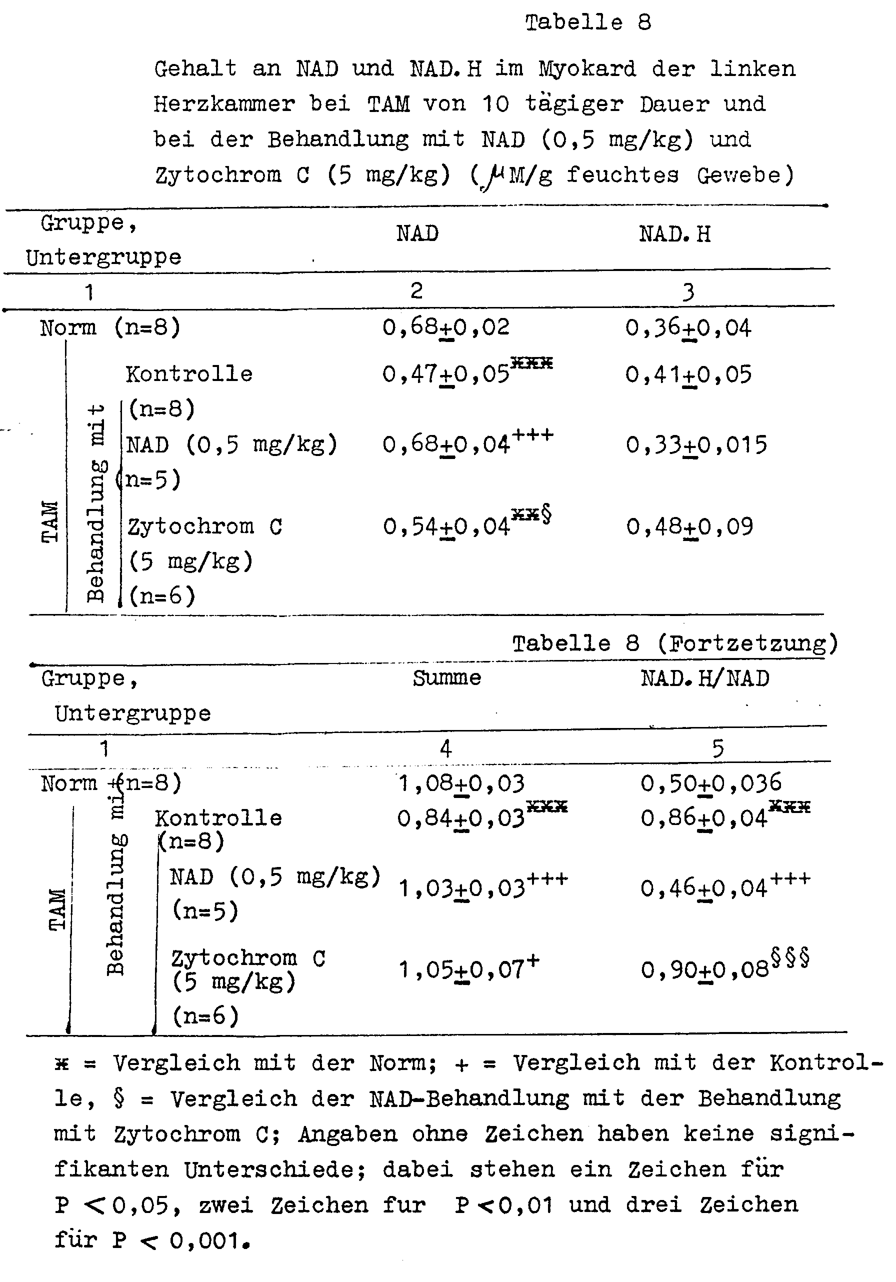

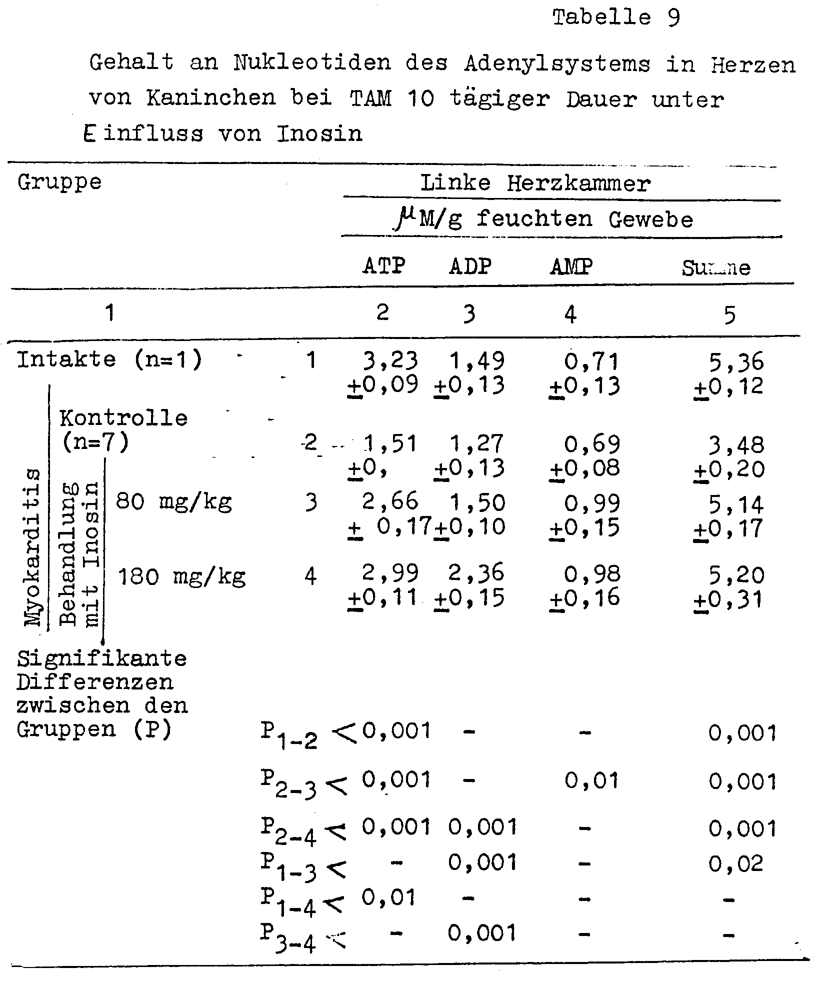

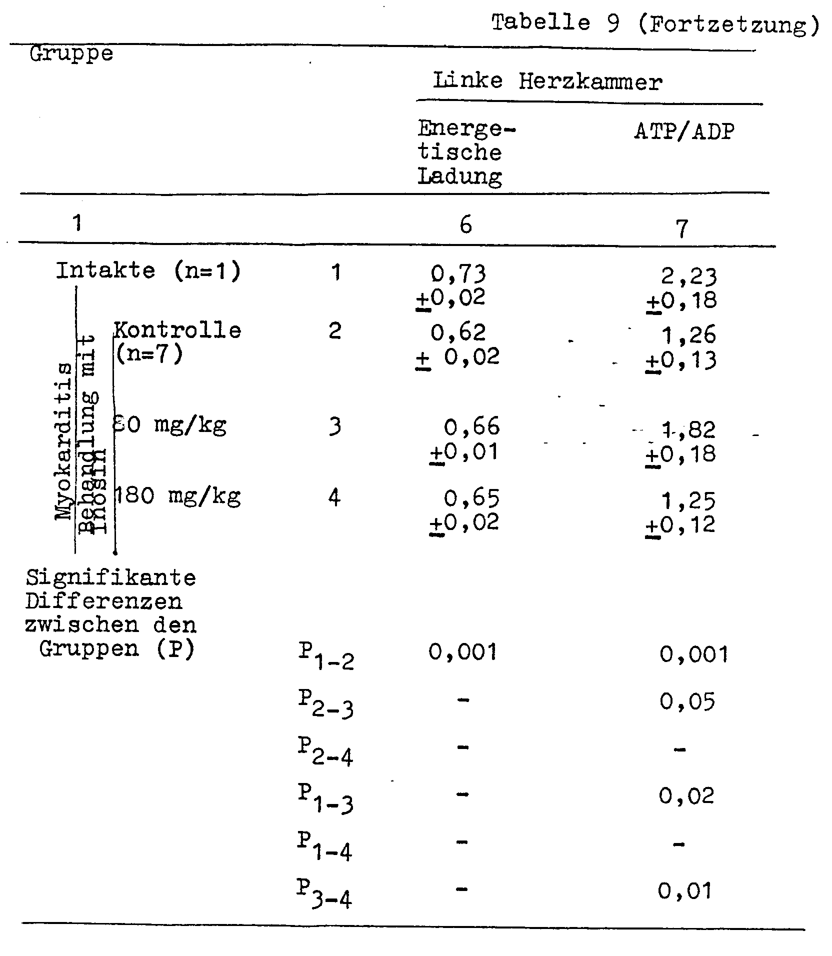

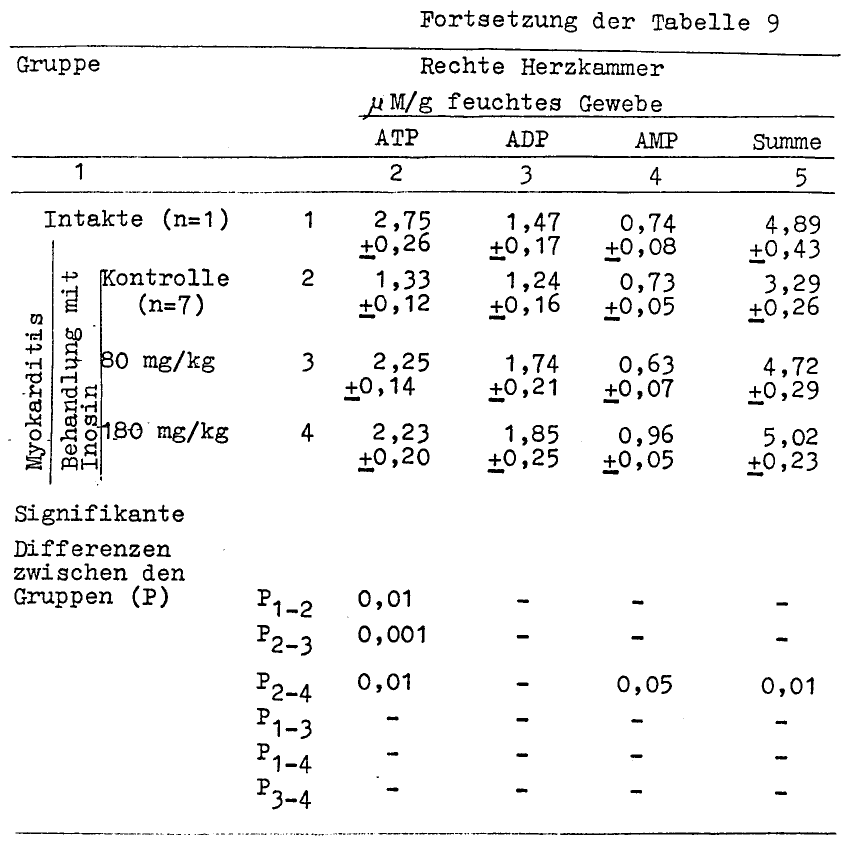

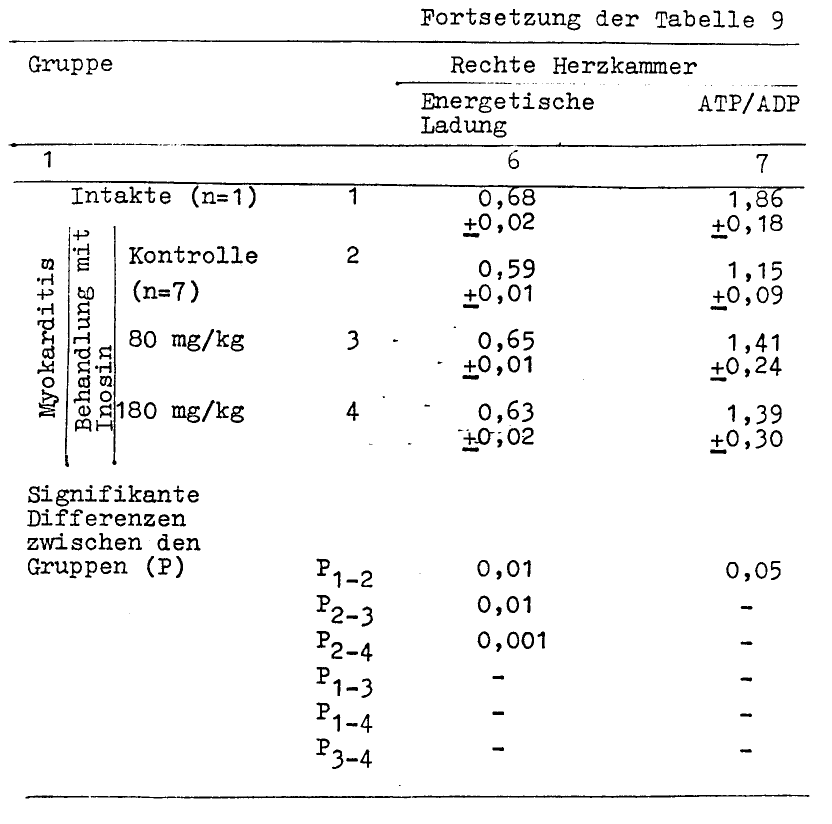

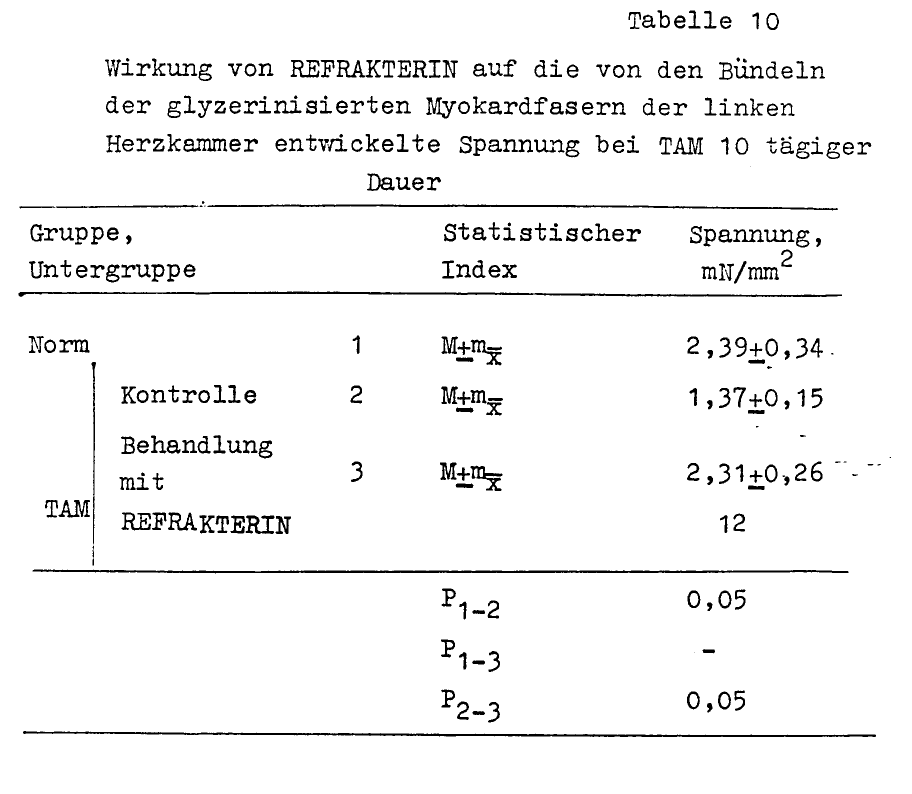

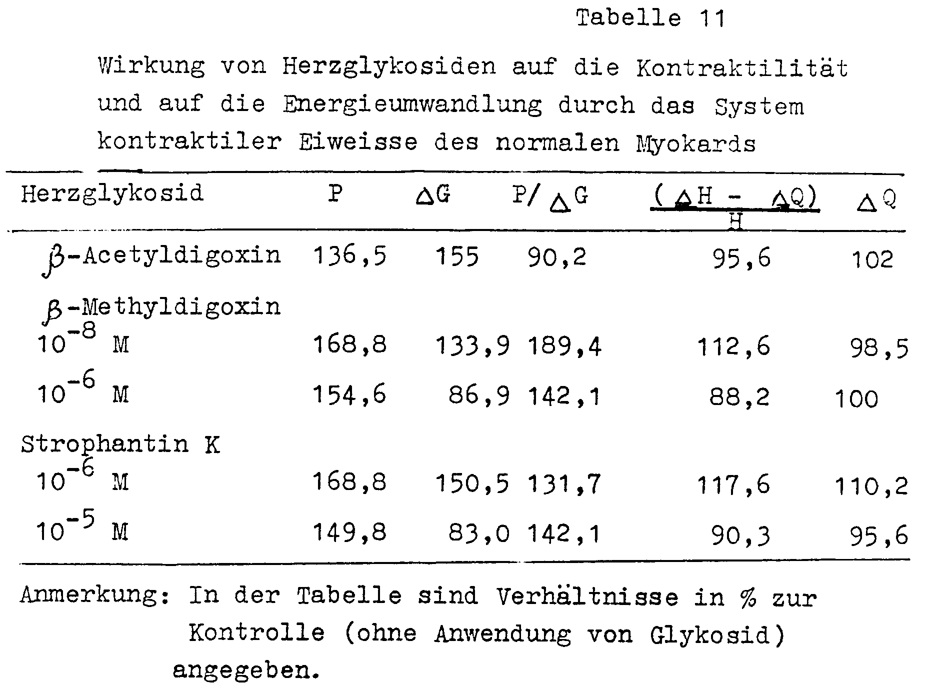

**Tabelle 12**

| Wirkung von Herzglykosiden auf die mechanischen und thermodynamischen Parameter des Kontraktionsvorganges bei TAM | | | |
|---|---|---|---|
| Herzglykosid | P | ΔH | ΔH - ΔQ/ ΔH |
| Kontrolle (10 tägige TAM) | 43,4 | 52,5 | 77,9 |
| β-Acetyldigoxin 10⁻⁶ M | 75,6 | 63,0 | 76,2 |
| Strophantin K 10⁻⁶ M | 75,6 | 65,7 | 94,1 |
| Anmerkung: In der Tabelle sind Verhältnisse in % zur Kontrolle (Norm) angegeben. | | | |

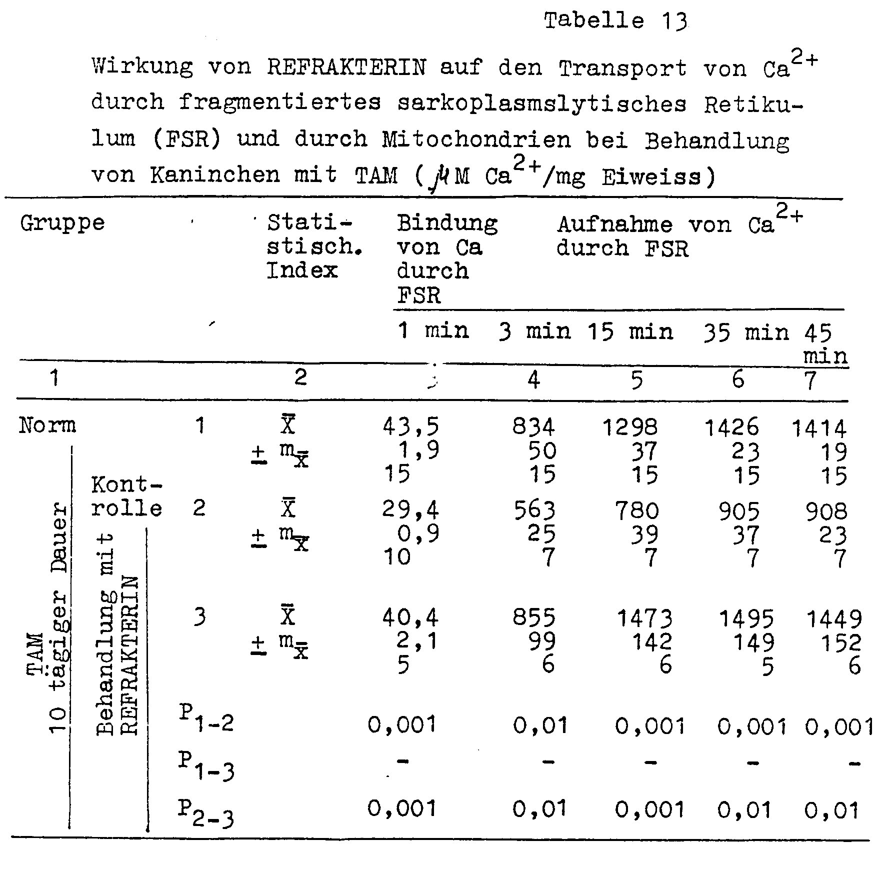

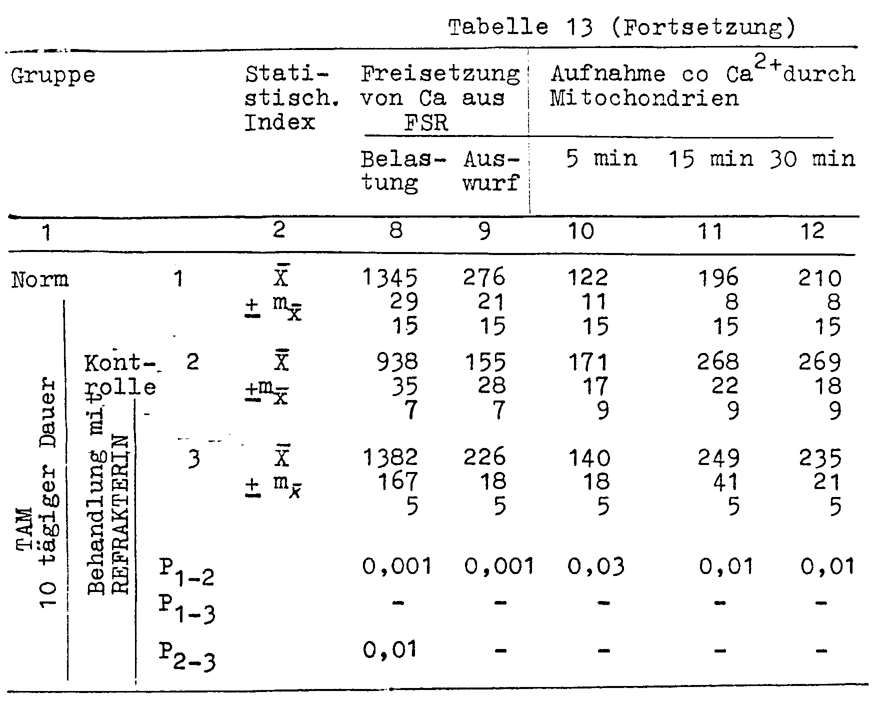

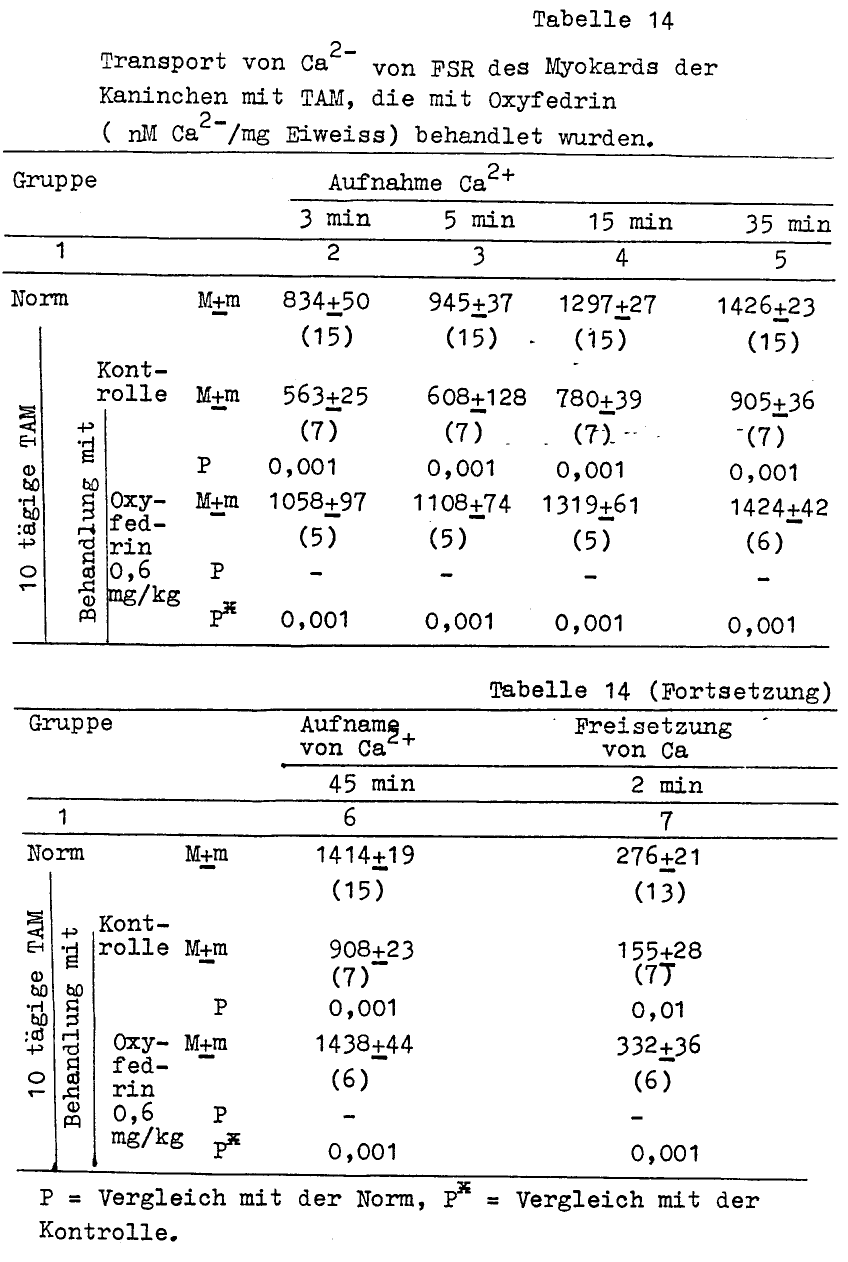

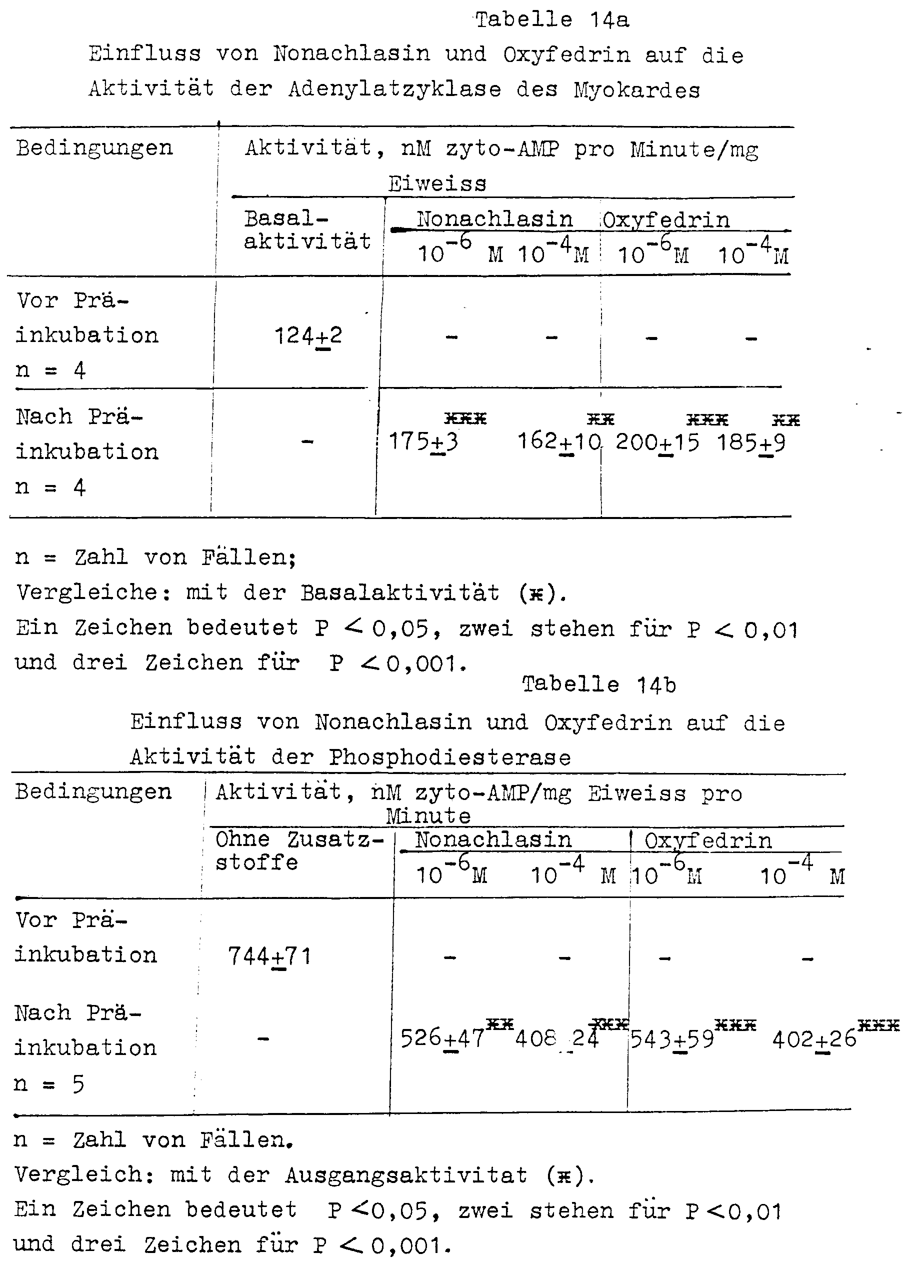

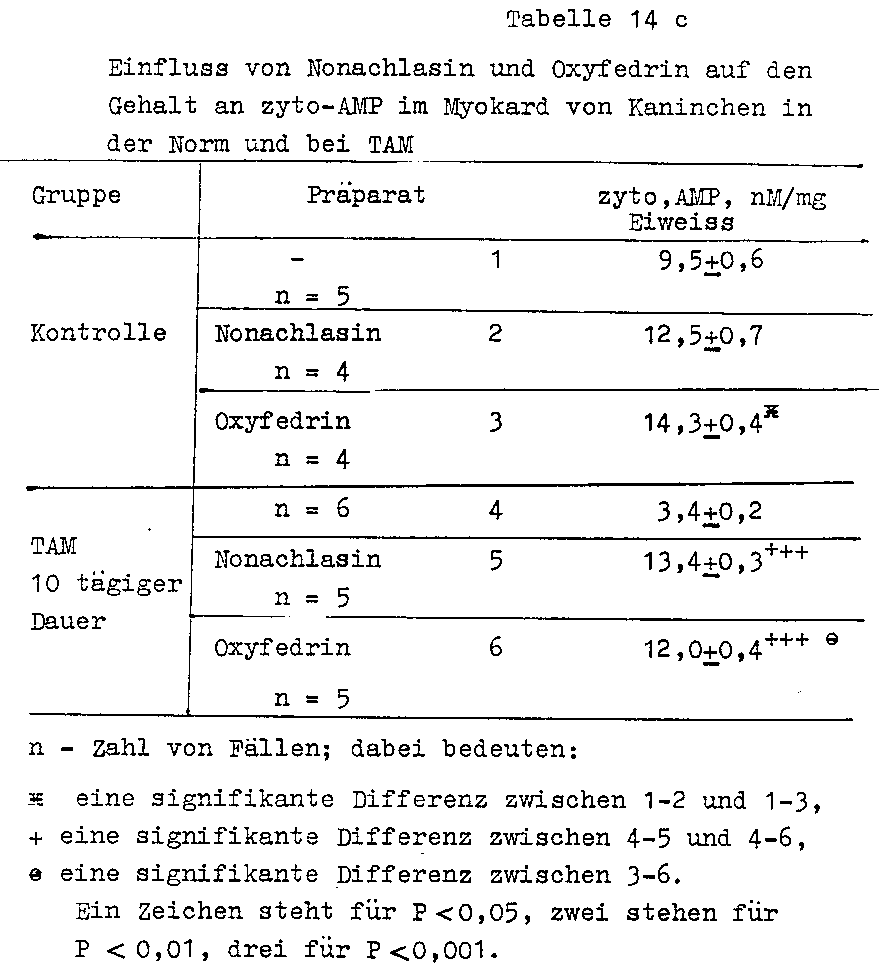

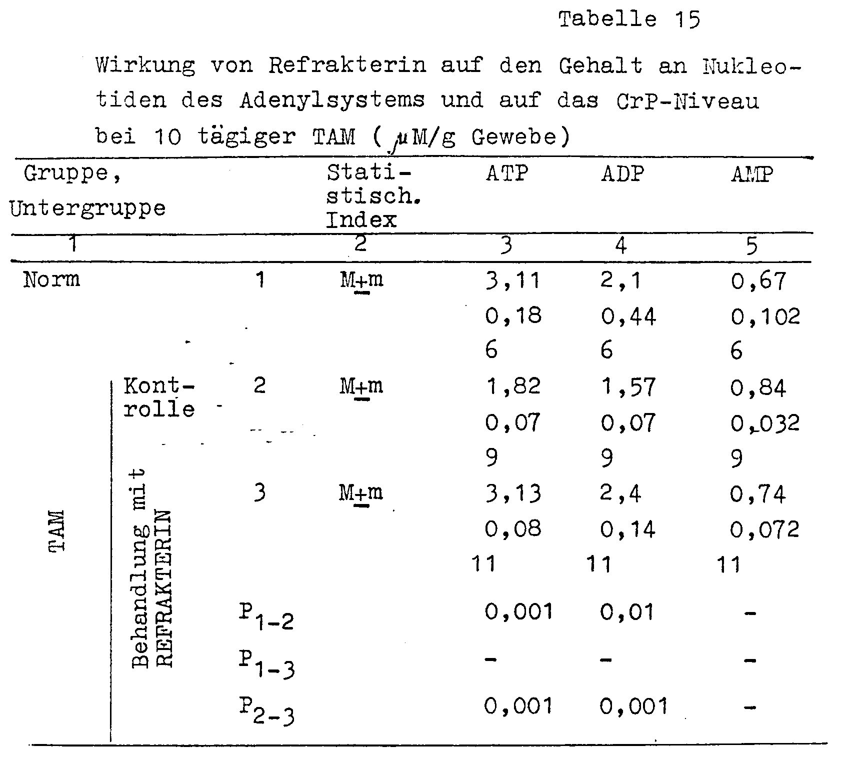

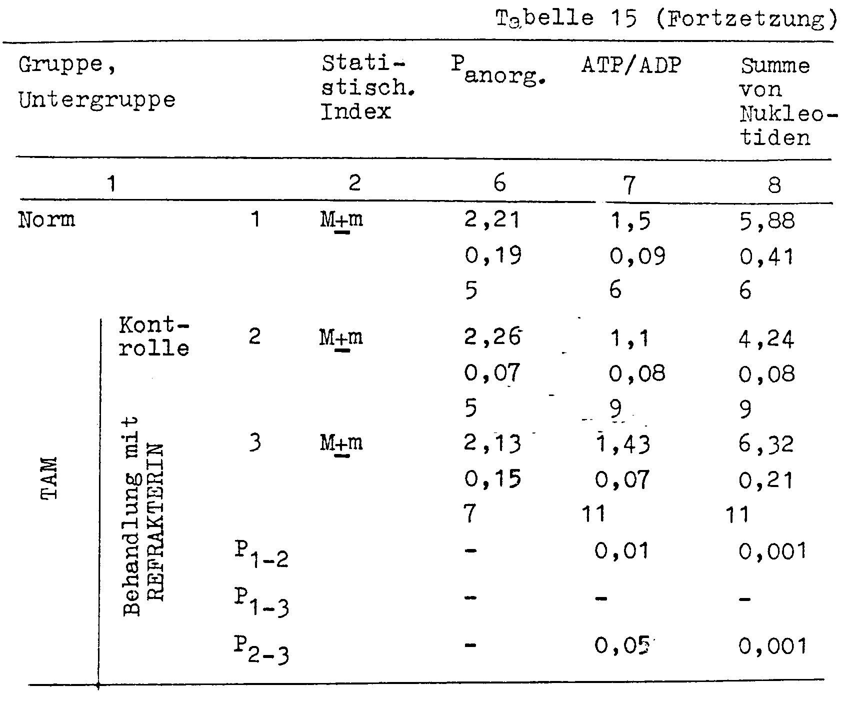

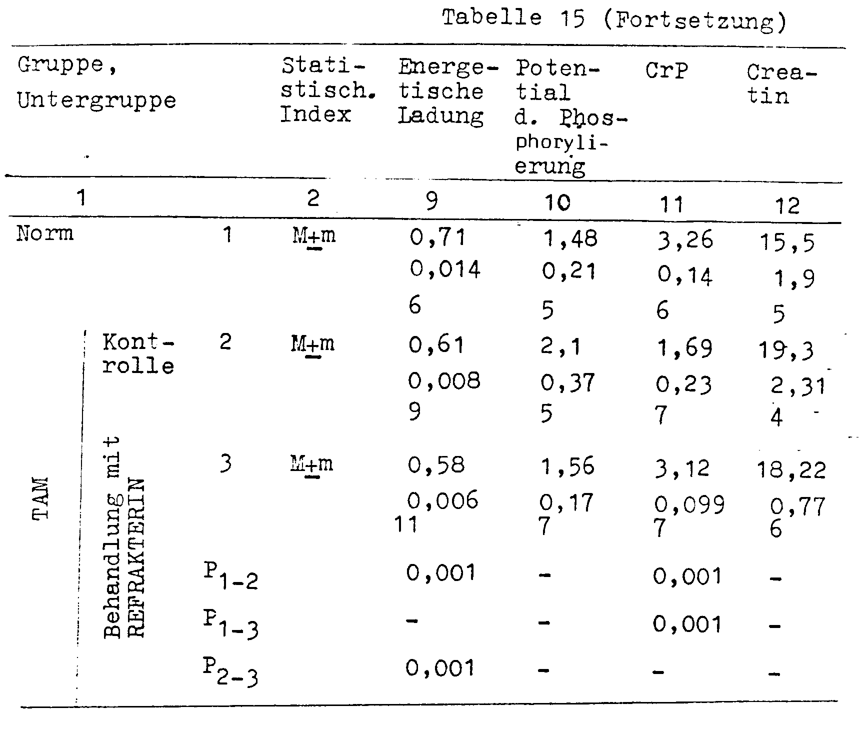

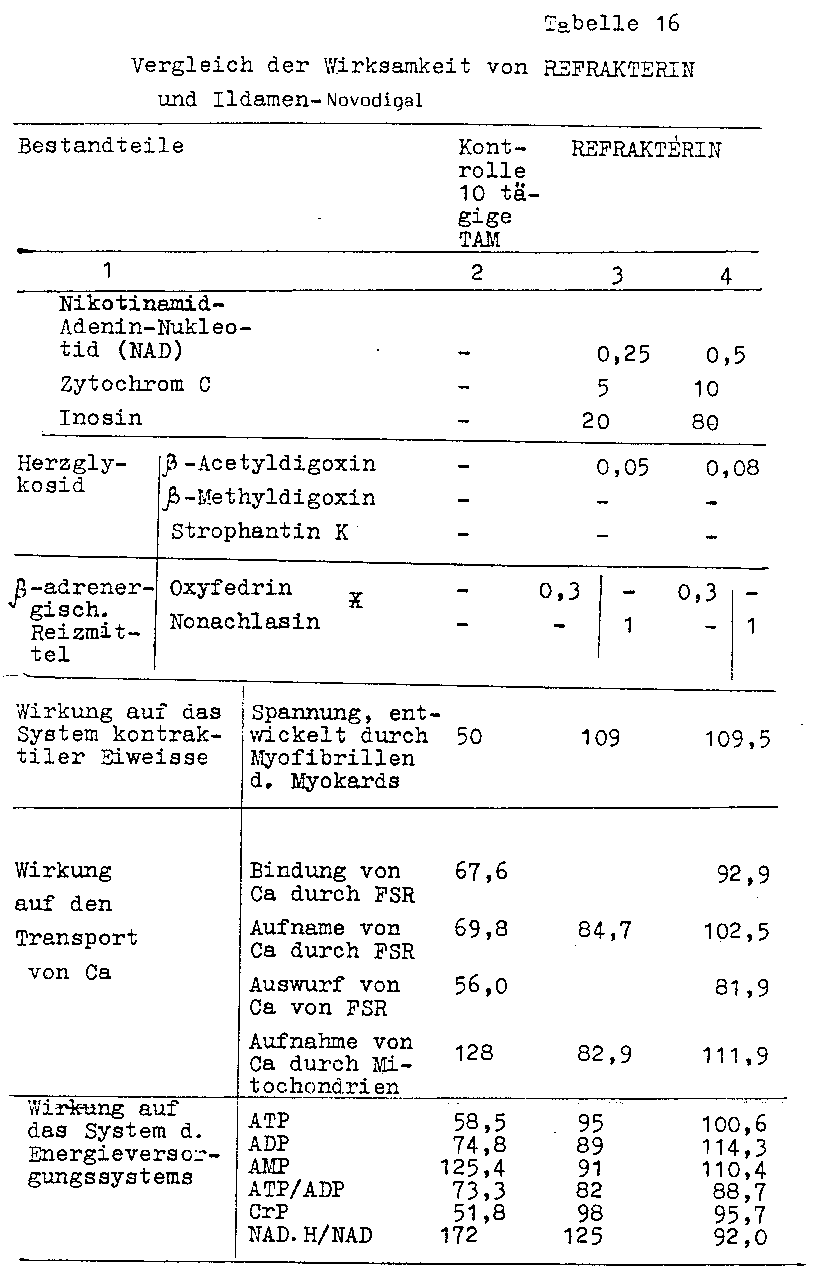

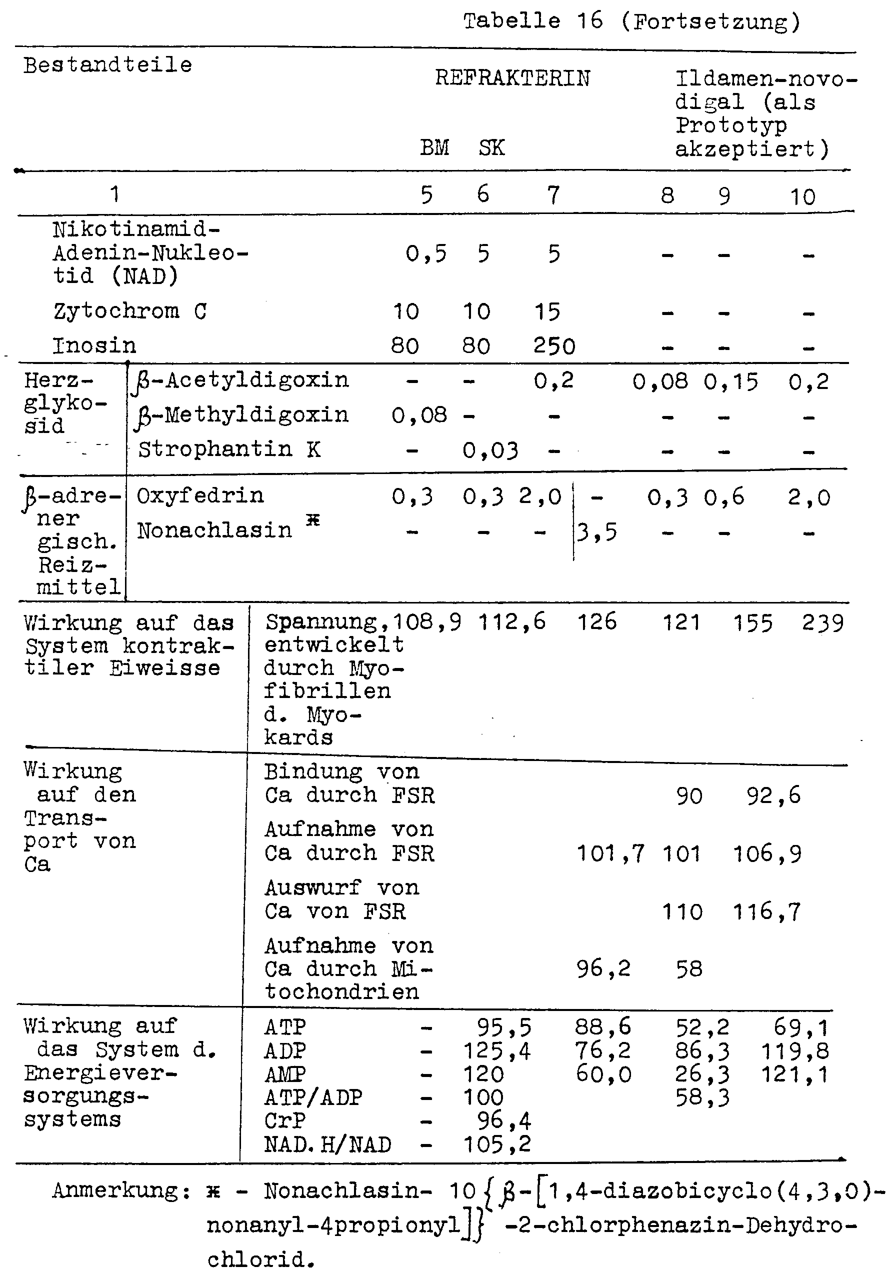

## Patentansprüche

1. Kardiotropes Mittel auf der Basis von Herzglykosid und einem β-Sympathomimetikum mit kardiotroper Wirkung, **dadurch gekennzeichnet**, daß es zusätzlich Nikotinamid-adenin-dinukleotid, Zytochrom C und Inosin bei folgendem Verhältnis der Bestandteile (in Gewichtsteilen) enthält:
| | |
|---|---|
| Herzglykosid | 0,05 bis 0,2 |
| β-Sympathomimetikum | 0,3 bis 3,5 |
| Nikotinamid-adenin-dinukleotid | 0,5 bis 5 |
| Zytochrom C | 5 bis 15 |
| Inosin | 20 bis 250 |

2. Kardiotropes Mittel nach Anspruch 1, **dadurch gekennzeichnet,** dass als Herzglykosid β-Acetyldigoxin in einer Menge von 0,05 bis 0,2 (Gewichtsteilen) benutzt ist.

3. Kardiotropes Mittel nach Anspruch 1, **dadurch gekennzeichnet,** dass man als Herzglykosid β-Methyldigoxin in einer Menge von 0,05 bis 0,15 (Gewichtsteilen) verwendet.

4. Kardiotropes Mittel nach Anspruch 1, **dadurch gekennzeichnet,** dass man als Herzglykosid Strophantin K in einer Menge von 0,05 bis 0,2 (Gewichtsteilen) verwendet.

5. Kardiotropes Mittel nach Anspruch 1, **dadurch gekennzeichnet**, daß man als β-Sympathomimetikum Oxyfedrin in einer Menge von 0,3 bis 2 (Gewichtsteilen) verwendet.

6. Kardiotropes Mittel nach Anspruch 1, **dadurch gekennzeichnet**, daß man als β-Sympathomimetikum Nonachlasin in einer Menge von 1 bis 3,5 (Gewichtsteilen) verwendet.

## Claims

1. Cardiotrophic composition based on cardiac glycoside and a β-sympathomimetic with a cardiotrophic effect, characterized in that it additionally contains nicotinamide adenine dinucleotide, cytochrome C and inosine with the following ratio of components (in parts by weight):
| | |
|---|---|
| cardiac glycoside | 0.05 to 0.2 |
| β-sympathomimetic | 0.3 to 3.5 |
| nicotinamide adenine dinucleotide | 0.5 to 5 |
| cytochrome C | 5 to 15 |
| inosine | 20 to 250 |

2. Cardiotrophic composition according to claim 1, characterized in that β-acetyl digoxin is used in a quantity of 0.05 to 0.2 (parts by weight) as cardiac glycoside.

3. Cardiotrophic composition according to claim 1, characterized in that β-methyl digoxin is used in a quantity of 0.05 to 0.15 (parts by weight) as cardiac glycoside.

4. Cardiotrophic composition according to claim 1, characterized in that strophanthin K is used in a quantity of 0.05 to 0.2 (parts by weight) as cardiac glycoside.

5. Cardiotrophic composition according to claim 1, characterized in that oxyphedrine is used in a quantity of 0.3 to 2 (parts by weight) as β-sympathomimetic.

6. Cardiotrophic composition according to claim 1, characterized in that nonachlasine is used in a quantity of 1 to 3.5 (parts by weight) as β-sympathomimetic.

## Revendications

1. Agent cardiotrope à base de glucoside cardiotonique et d'un β-sympathomimétique à effet cardiotrope, caractérisé en ce qu'il contient en outre du nicotinamide-adénine-dinucléotide, du cytochrome C et de l'inosine dans les proportions suivantes des constituants (en parties en masse) :
| | |
|---|---|
| glucoside cardiotonique | 0,05 à 0,2 |
| β-sympathomimétique | 0,3 à 3,5 |
| nicotinamide-adénine-dinucléotide | 0,5 à 5 |
| cytochrome C | 5 à 15 |
| inosine | 20 à 250 |

2. Agent cardiotrope selon la revendication 1, caractérisé en ce que l'on utilise comme glucoside cardiotonique la β-acétyldigoxine en une quantité de 0,05 à 0,2 (parties en masse).

3. Agent cardiotrope selon la revendication 1, caractérisé en ce que l'on utilise comme glucoside cardiotonique la β-méthyldigoxine en une quantité de 0,05 à 0,15 (parties en masse).

4. Agent cardiotrope selon la revendication 1, caractérisé en ce que l'on utilise comme glucoside cardiotonique la strophantine K en une quantité de 0,05 à 0,2 (parties en masse).

5. Agent cardiotrope selon la revendication 1, caractérisé en ce que l'on utilise comme β-sympathomimétique l'oxyfédrine en une quantité de 0,3 à 2 (parties en masse).

6. Agent cardiotrope selon la revendication 1, caractérisé en ce que l'on utilise comme β-sympathomimétique la nonachlasine en une quantité de 1 à 3,5 (parties en masse).
